(19) **Europäisches Patentamt / European Patent Office / Office européen des brevets**

(11) **EP 3 858 344 A1**

(12) # EUROPEAN PATENT APPLICATION
published in accordance with Art. 153(4) EPC

(43) Date of publication:
04.08.2021 Bulletin 2021/31

(21) Application number: 19867502.7

(22) Date of filing: 26.09.2019

(51) Int Cl.:
*A61K 31/282* (2006.01)  *A61K 31/513* (2006.01)
*A61K 31/519* (2006.01)  *A61K 39/395* (2006.01)
*A61P 35/00* (2006.01)  *A61P 43/00* (2006.01)
*G01N 33/50* (2006.01)  *G01N 33/68* (2006.01)

(86) International application number:
**PCT/JP2019/037733**

(87) International publication number:
**WO 2020/067228 (02.04.2020 Gazette 2020/14)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 28.09.2018  JP 2018185142

(71) Applicants:
• **Keio University**
  **Tokyo, 108-8345 (JP)**
• **Kabushiki Kaisha Yakult Honsha**
  **Tokyo, 105-8660 (JP)**

(72) Inventors:
• **SUGIMOTO, Shinji**
  **Tokyo 160-8582 (JP)**
• **TANIGAWARA, Yusuke**
  **Tokyo 160-8582 (JP)**
• **MATSUO, Mitsuhisa**
  **Tokyo 105-8660 (JP)**
• **TAKAHASHI, Hiroyuki**
  **Tokyo 105-8660 (JP)**

(74) Representative: **Schiener, Jens**
  **Wächtershäuser & Hartz**
  **Patentanwaltspartnerschaft mbB**
  **Weinstraße 8**
  **80333 München (DE)**

(54) **MARKER FOR ASSESSING SENSITIVITY TO COMBINATION ANTICANCER DRUG**

(57)    Provided is a marker for determining sensitivity to an anti-cancer agent early after the start of treatment with the anti-cancer agent. The present invention provides a marker for determining sensitivity to an anti-cancer agent early after the start of treatment with the anti-cancer agent, the anti-cancer agent comprising oxaliplatin or a salt thereof, fluorouracil or a salt thereof, and levofolinate or a salt thereof, the marker comprising one or more molecules selected from the group consisting of 2AMAD, 2ABA, 2CYPR, 50PRO, 6AHXA, ADEN, ASP, BETNC, CARB, CSSG, DOPM, GGLCY, GSSG, HYPT, METSF, N6MDA, NOMTR, PHEP, PRO and RIB5P.

EP 3 858 344 A1

**Description**

Technical Field

The present invention relates to a marker for determining sensitivity to an anti-cancer agent which is used to determine, early after the start of treatment with the anti-cancer agent, whether or not a cancer of a patient of interest has a therapeutic response to the anti-cancer agent, and to use of the marker.

Background Art

Anti-cancer agents include various types of agents such as an alkylating agent, a platinum agent, an antimetabolite, an anti-cancer antibiotic, and an anti-cancer plant alkaloid. These anti-cancer agents are effective for some types of cancers but not effective for other types of cancers. However, it is known that even when an anti-cancer agent has been confirmed to be effective for a certain type of cancer, the anti-cancer agent is effective for some patients and not effective for other patients, leading to interindividual differences. Whether or not a cancer of a patient has a response to an anti-cancer agent is designated as sensitivity to the anti-cancer agent.

Oxaliplatin, (SP-4-2)-[(1R,2R)-cyclohexane-1,2-diamine-KN,KN'] [ethanedioato(2-)-KO$^1$,KO$^2$]platinum (IUPAC), is a third generation platinum-based complex anti-neoplastic agent. The action mechanism thereof is thought to be based on inhibition of DNA synthesis or protein synthesis via cross-linking with DNA bases, similar to precedent platinum-based complex anti-cancer agents such as cisplatin (CDDP) and carboplatin (CBDCA). However, oxaliplatin (L-OHP) exhibits an anti-tumor effect even against colorectal cancer, while CDDP or CBDCA does not, thus oxaliplatin shows different anti-tumor spectrum from the precedent platinum-based complex anti-neoplastic agents. In the United States, oxaliplatin, in combination with fluorouracil (5-FU)/levofolinate (LV), was approved as a first line therapy for metastatic colorectal cancer in January, 2004. In Japan, oxaliplatin was listed in the National Health Insurance price list in April, 2005 for "advanced/recurrent colorectal cancer not amenable to curative surgical resection", as a combination with other anti-neoplastic agents (the usefulness is acknowledged in the case of combination of oxaliplatin with continuous intravenous infusion of levofolinate and fluorouracil and the like). For advanced/recurrent colorectal cancer, 5-FU/LV regimen, which had been employed until early 1990's, provided a survival of 10 to 12 months. In contrast, FOLFOX regimen, which further comprises oxaliplatin, has achieved a survival period of 19.5 months which is almost twice of that of the 5-FU/LV regimen. In August, 2009, the combined use of oxaliplatin with continuous intravenous infusion of levofolinate/fluorouracil was also listed for "postoperative adjuvant chemotherapy for colon cancer", which was added as another efficacy and effectiveness. Thus, oxaliplatin is a drug that is promising for extended use and benefits in colorectal cancer patients. Besides colorectal cancer, the efficacy and effectiveness were added for unresectable pancreatic cancer in August, 2009 and for gastric cancer in March, 2015, as a combination of oxaliplatin with other chemotherapeutic agents.

However, even so, the response rate of FOLFOX therapy against advanced/recurrent colorectal cancer is about 55%. In other words, the primary therapy is effective for only half of patients who have received the therapy and the secondary therapy is effective for only one in five to ten patients. Also, use of oxaliplatin may cause neutropenia as well as severe diarrhea, resulting in fatal outcome in some cases. If there is a biomarker which can predict, before starting the therapy, which patients can expect to have an efficacy and which patients cannot, and can diagnose therapeutic response in an early stage of the therapy, a chemotherapy treatment with high effectiveness and high safety can be realized.

Furthermore, since a therapy schedule of cancer chemotherapy generally takes a long period of time, monitoring over time of sensitivity to an anti-cancer agent during the therapy enables determination of whether the therapy should be continued. Not only this leads to reduction of patient's burden or adverse effects, but this is also considered useful even from the viewpoint of medical economics. In order to realize a "personalized therapy" which predicts therapeutic response in individual patients and diagnoses in an early stage to select an appropriate medicament or therapeutic regimen, it is urgently needed to establish a biomarker which enables prediction of the efficacy of an anti-cancer agent such as oxaliplatin or an early diagnosis of therapeutic response, is urgent.

From such viewpoints, the present inventors conducted a comprehensive analysis of intracellular metabolic variation in multiple human cancer cell lines having different drug sensitivity or in cancer-bearing mice transplanted with the human cancer cell lines after they had been exposed to drugs, using capillary electrophoresis time-of-flight mass spectrometer (CE-TOF MS). Then, the inventors conducted a comparative analysis of the results with drug sensitivity to search for a marker for determining sensitivity to an anti-cancer agent, and reported the obtained several markers (Patent Literatures 1 to 4). However, these markers have not yet been put into practical use. Furthermore, combination therapies of FOLFOX regimen with an antibody medicine such as bevacizumab, cetuximab or panitumumab have been recently established. Thus, there is an increasing importance of a biomarker which enables to predict an effect of FOLFOX regimen or to diagnose a therapeutic response in an early stage of the therapy.

Citation List

Patent Literature

Patent Literature 1: WO 2009/096189
Patent Literature 2: WO 2011/052750
Patent Literature 3: WO 2012/127984
Patent Literature 4: WO 2013/125675

Summary of the Invention

Problems to be Solved by the Invention

An object of the present invention is to provide a marker for determining sensitivity to an anti-cancer agent, which can determine a therapeutic response of each individual patient early after the start of treatment.

Means for Solving the Problems

Accordingly, the present inventors comprehensively measured blood metabolites in blood specimens from colorectal cancer patients after implementation of one cycle or after implementation of two cycles of bevacizumab plus mFOLFOX6 therapy using CE-Q-TOF MS and CE-TOF MS, and conducted logistic analysis by using the obtained concentrations of the metabolites as explanatory variables and the clinical effects as objective variables. As a result, the present inventors found that: the concentrations of particular substances differ between patients of a responder group having high therapeutic response to bevacizumab plus mFOLFOX6 therapy and patients of a non-responder group having low therapeutic response thereto; and these substances are useful as markers for determining sensitivity early after the start of treatment with an anti-cancer agent. The present inventors also conducted proportional hazard model analysis on the blood metabolites of the cancer patients after implementation of one cycle and residual overall survivals, or the blood metabolites after implementation of two cycles and residual overall survivals. As a result, the present inventors found that: higher blood concentrations of particular substances lead to a longer survival period while higher blood concentrations of other particular substances lead to a shorter survival period; and these substances are useful as markers for predicting prognosis early after the start of treatment with an anti-cancer agent. On the basis of these findings, the present invention has been completed.

Specifically, the present invention provides aspects of the following [1] to [15].

[1] A marker for determining sensitivity to an anti-cancer agent early after the start of treatment with the anti-cancer agent, the anti-cancer agent comprising oxaliplatin or a salt thereof, fluorouracil or a salt thereof, and levofolinate or a salt thereof, the marker comprising one or more molecules selected from the group consisting of 2-aminoadipic acid (2AMAD), 2-aminobutyric acid (2ABA), 2-cyanopyridine (2CYPR), 5-oxoproline (5OPRO), 6-aminohexanoic acid (6AHXA), adenosine (ADEN), aspartic acid (ASP), betonicine (BETNC), carbachol (CARB), cysteine-glutathione disulphide (CSSG), dopamine (DOPM), gamma-glutamylcysteine (GGLCY), oxidized glutathione (GSSG), hypotaurine (HYPT), methionine sulfoxide (METSF), N6-methyl-2'-deoxyadenosine (N6MDA), N-omega-methyltryptamine (NOMTR), phenyl phosphate (PHEP), proline (PRO) and ribulose 5-phosphate (RIB5P).
[2] The marker according to [1], wherein the anti-cancer agent further comprises bevacizumab.
[3] A method for determining sensitivity to an anti-cancer agent early after the start of treatment with the anti-cancer agent, the anti-cancer agent comprising oxaliplatin or a salt thereof, fluorouracil or a salt thereof, and levofolinate or a salt thereof, the method comprising measuring an amount of one or more molecules selected from the group consisting of 2AMAD, 2ABA, 2CYPR, 5OPRO, 6AHXA, ADEN, ASP, BETNC, CARB, CSSG, DOPM, GGLCY, GSSG, HYPT, METSF, N6MDA, NOMTR, PHEP, PRO and RIB5P in a biological sample derived from a cancer patient who has received at least one cycle of treatment with the anti-cancer agent comprising oxaliplatin or a salt thereof, fluorouracil or a salt thereof, and levofolinate or a salt thereof.
[4] The determination method according to [3], further comprising determining the sensitivity of the cancer patient to the anti-cancer agent by comparing a measurement result with a control level.
[5] The determination method according to [4], wherein: the cancer patient is a patient who has received one cycle of treatment with the anti-cancer agent; the amount of one or more molecules selected from the group consisting of 2AMAD, 2CYPR, 6AHXA, BETNC, CARB, CSSG, GGLCY, GSSG, N6MDA, NOMTR, PHEP and RIB5P is measured; the control level is a cutoff value of a responder; and the cutoff value is $2.091 \times 10^{-2} \leq$ for 2AMAD, $3.444 \times$

$10^{-3} \leq$ for 2CYPR, $7.175 \times 10^{-3} \leq$ for 6AHXA, $1.589 \times 10^{-2} \leq$ for BETNC, $\leq 8.472 \times 10^{-3}$ for CARB, $2.198 \times 10^{-2} \leq$ for CSSG, $7.389 \times 10^{-3} \leq$ for GGLCY, $5.606 \times 10^{-4} \leq$ for GSSG, $\leq 1.208 \times 10^{-3}$ for N6MDA, $\leq 7.006 \times 10^{-2}$ for NOMTR, $\leq 1.801 \times 10^{-2}$ for PHEP, and $\leq 3.005 \times 10^{-3}$ for RIB5P.

[6] The determination method according to [3], wherein the cancer patient is a patient who has received one cycle of treatment with the anti-cancer agent, and the method further comprises calculating a probability (p) of a responder according to the following equation (1) to determine whether or not the cancer patient is the responder:

$$p = \frac{1}{1 + e^{(-16.4681 + (2\text{AMAD}) + (\text{BETNC}) + (\text{CSSG}) + (\text{N6MDA}) + (\text{RIB5P}))}} \quad (1)$$

wherein 2AMAD represents -16.2688 when a measurement result about 2AMAD is equal to or more than a cutoff value, and 16.2688 when the measurement result is less than the cutoff value; BETNC represents -8.6560 when a measurement result about BETNC is equal to or more than a cutoff value, and 8.6560 when the measurement result is less than the cutoff value; CSSG represents -1.4372 when a measurement result about CSSG is equal to or more than a cutoff value, and 1.4372 when the measurement result is less than the cutoff value; N6MDA represents -8.6658 when a measurement result about N6MDA is equal to or less than a cutoff value, and 8.6658 when the measurement result is more than the cutoff value; RIB5P represents -1.3451 when a measurement result about RIB5P is equal to or less than a cutoff value, and 1.3451 when the measurement result is more than the cutoff value; and the cutoff value is $2.091 \times 10^{-2}$ for 2AMAD, $1.589 \times 10^{-2}$ for BETNC, $2.198 \times 10^{-2}$ for CSSG, $1.208 \times 10^{-3}$ for N6MDA, and $3.005 \times 10^{-3}$ for RIB5P.

[7] The determination method according to [4], wherein the cancer patient is a patient who has received two cycles of treatment with the anti-cancer agent; the amount of one or more molecules selected from the group consisting of 2ABA, 5OPRO, 6AHXA, ADEN, ASP, DOPM, HYPT, METSF and PRO is measured; the control level is a cutoff value of a responder; and the cutoff value is $2.507 \times 10^{-1} \leq$ for 2ABA, $1.843 \times 10^{-1} \leq$ for 5OPRO, $4.568 \times 10^{-3} \leq$ for 6AHXA, $2.305 \times 10^{-2} \leq$ for ADEN, $\leq 1.170 \times 10^{-1}$ for ASP, $5.606 \times 10^{-4} \leq$ for DOPM, $\leq 1.768 \times 10^{-2}$ for HYPT, $3.836 \times 10^{-2} \leq$ for METSF, and $2.9876 \leq$ for PRO.

[8] The determination method according to [3], wherein the cancer patient is a patient who has received two cycles of treatment with the anti-cancer agent, and the method further comprises calculating a probability (p) of a responder according to the following equation (2) to determine whether or not the cancer patient is the responder:

$$p = \frac{1}{1 + e^{(6.4305 + (2\text{ABA}) + (\text{ASP}) + (\text{DOPM}) + (\text{HYPT}) + (\text{METSF}) + (\text{PRO}))}} \quad (2)$$

wherein 2ABA represents -2.3059 when a measurement result about 2ABA is equal to or more than a cutoff value, and 2.3059 when the measurement result is less than the cutoff value; ASP represents -1.8154 when a measurement result about ASP is equal to or less than a cutoff value, and 1.8154 when the measurement result is more than the cutoff value; DOPM represents -1.6345 when a measurement result about DOPM is equal to or more than a cutoff value, and 1.6345 when the measurement result is less than the cutoff value; HYPT represents -2.4113 when a measurement result about HYPT is equal to or less than a cutoff value, and 2.4113 when the measurement result is more than the cutoff value; METSF represents -1.5555 when a measurement result about METSF is equal to or more than a cutoff value, and 1.5555 when the measurement result is less than the cutoff value; PRO represents -9.0794 when a measurement result about PRO is equal to or more than a cutoff value, and 9.0794 when the measurement result is less than the cutoff value; and the cutoff value is $2.507 \times 10^{-1}$ for 2ABA, $1.170 \times 10^{-1}$ for ASP, $5.606 \times 10^{-4}$ for DOPM, $1.768 \times 10^{-2}$ for HYPT, $3.836 \times 10^{-2}$ for METSF, and 2.9876 for PRO.

[9] The determination method according to any one of [3] to [8], wherein the anti-cancer agent further comprises bevacizumab.

[10] A marker for predicting prognosis early after the start of treatment with an anticancer agent, the anti-cancer agent comprising oxaliplatin or a salt thereof, fluorouracil or a salt thereof, and levofolinate or a salt thereof, the marker comprising one or more molecules selected from the group consisting of 10-hydroxydecanoic acid (10HDA), 2AMAD, alanine (ALA), benzimidazole (BEZIZ), cholic acid (CHOA), CSSG, dodecanedioic acid (DDNA), GGLCY, gluconic acid (GLCOA), glutamic acid (GLU), glutaric acid (GLTA), glycerol-3-phosphate (GLC3P), glycylleucine (GLYLE), guanidinosuccinic acid (GUSA), leucine (LEU), lysine (LYS), N6-acetyllysine (N6ALY), octanoic acid (OCTA), pipecolic acid (PIPEC), quinic acid (QUINA), threonine (THR) and valine (VAL).

[11] The marker according to [10], wherein the anti-cancer agent further comprises bevacizumab.

[12] A method for predicting prognosis early after the start of treatment with an anti-cancer agent, the anti-cancer agent comprising oxaliplatin or a salt thereof, fluorouracil or a salt thereof, and levofolinate or a salt thereof, the method comprising measuring an amount of one or more molecules selected from the group consisting of 10HDA, 2AMAD, ALA, BEZIZ, CHOA, CSSG, DDNA, GGLCY, GLCOA, GLU, GLTA, GLC3P, GLYLE, GUSA, LEU, LYS, N6ALY, OCTA, PIPEC, QUINA, THR and VAL in a biological sample derived from a cancer patient who has received at least one cycle of treatment with the anti-cancer agent comprising oxaliplatin or a salt thereof, fluorouracil or a salt thereof, and levofolinate or a salt thereof.

[13] The prognosis prediction method according to [12], wherein the anti-cancer agent further comprises bevacizumab.

[14] A kit for carrying out a determination method according to any one of [3] to [9], the kit comprising a protocol for measuring an amount of one or more molecules selected from the group consisting of 2AMAD, 2ABA, 2CYPR, 5OPRO, 6AHXA, ADEN, ASP, BETNC, CARB, CSSG, DOPM, GGLCY, GSSG, HYPT, METSF, N6MDA, NOMTR, PHEP, PRO and RIB5P in a biological sample derived from a cancer patient who has received at least one cycle of treatment with the anti-cancer agent comprising oxaliplatin or a salt thereof, fluorouracil or a salt thereof, and levofolinate or a salt thereof.

[15] A kit for carrying out a prognosis prediction method according to [12] or [13], the kit comprising a protocol for measuring an amount of one or more molecules selected from the group consisting of 10HDA, 2AMAD, ALA, BEZIZ, CHOA, CSSG, DDNA, GGLCY, GLCOA, GLU, GLTA, GLC3P, GLYLE, GUSA, LEU, LYS, N6ALY, OCTA, PIPEC, QUINA, THR and VAL in a biological sample derived from a cancer patient who has received at least one cycle of treatment with the anti-cancer agent comprising oxaliplatin or a salt thereof, fluorouracil or a salt thereof, and levofolinate or a salt thereof.

Effects of the Invention

Use of the marker for determining sensitivity to an anti-cancer agent early after the start of treatment with the anti-cancer agent or the marker for predicting prognosis early after the start of treatment with the anti-cancer agent according to the present invention enables each individual patient to be evaluated for anti-cancer agent sensitivity or prognosis early after the start of treatment, and can determine whether or not to continue the treatment. As a result, the continuous administration of an anti-cancer agent having high therapeutic effects can be determined, whereas the continuous administration of an anti-cancer agent having no therapeutic effects can be circumvented. Thus, the progression of cancer and increase in adverse reactions expected by the continuous administration can be prevented, also leading to reduction in burdens on patients and reduction in medical cost. A reagent for measuring the marker for determining sensitivity to an anti-cancer agent early after the start of treatment with the anti-cancer agent or the marker for predicting prognosis early after the start of treatment with the anti-cancer agent according to the present invention is useful as a reagent for determining sensitivity to the anti-cancer agent early after the start of treatment with the anti-cancer agent or a reagent for predicting prognosis early after the start of treatment with the anti-cancer agent.

Brief Description of the Drawings

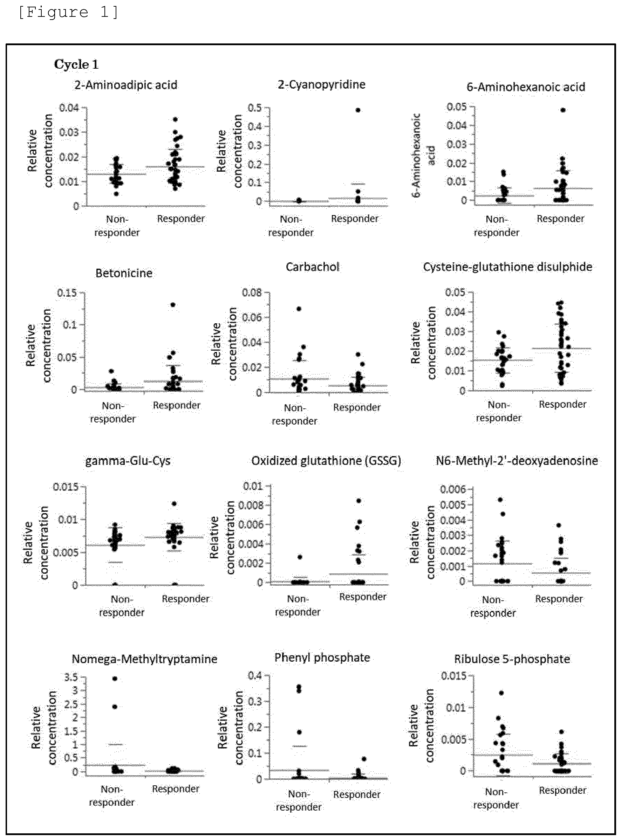

[Figure 1] Figure 1 is a diagram showing substances which exhibited significant difference in concentration after implementation of one cycle of treatment between a responder (R) group and a non-responder (N-R) group having distinct therapeutic response to bevacizumab plus mFOLFOX6 therapy.

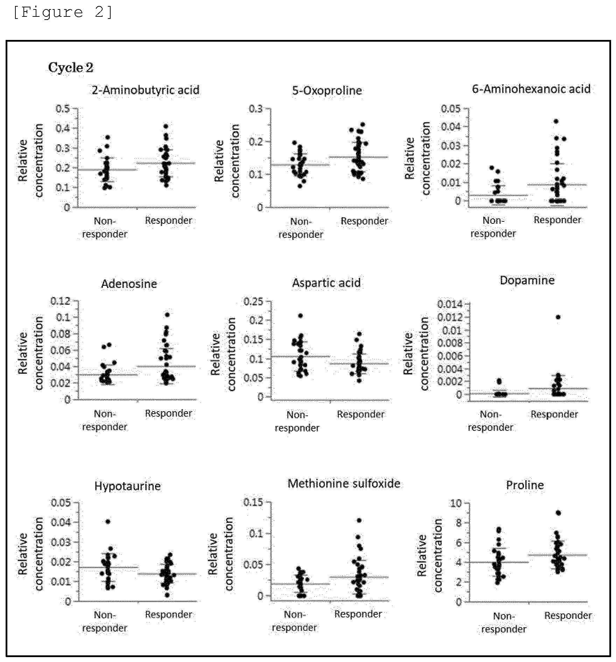

[Figure 2] Figure 2 is a diagram showing substances which exhibited significant difference in concentration after implementation of two cycles of treatment between a responder (R) group and a non-responder (N-R) group having distinct therapeutic response to bevacizumab plus mFOLFOX6 therapy.

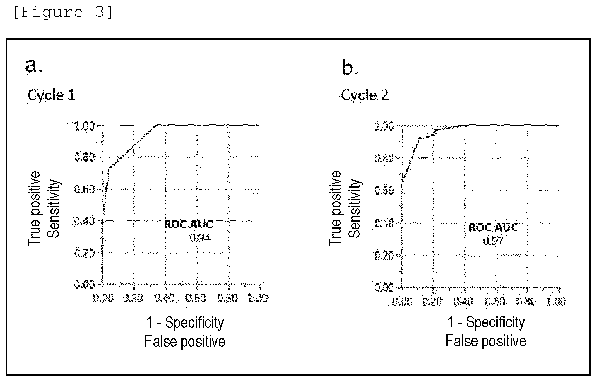

[Figure 3] Figure 3(a) is a diagram showing a ROC curve of a model for determining sensitivity to an anti-cancer agent early after the start of treatment with the anti-cancer agent according to the equation (1). Figure 3(b) is a diagram showing a ROC curve of a model for determining sensitivity to an anti-cancer agent early after the start of treatment with the anti-cancer agent according to the equation (2).

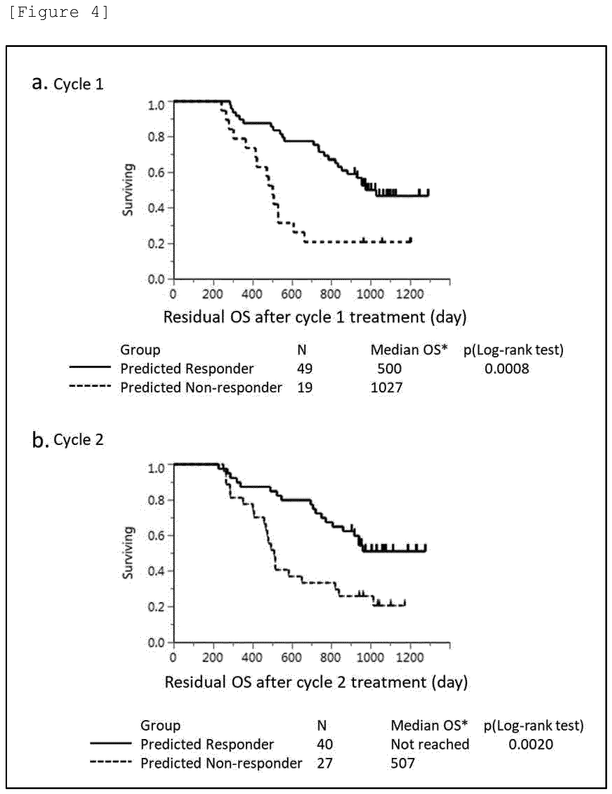

[Figure 4] Figure 4(a) is a diagram showing a Kaplan-Meier curve drawn after division of patients into a R group and a N-R group on the basis of metabolites after implementation of one cycle of treatment according to the equation (1). Figure 4(b) is a diagram showing a Kaplan-Meier curve drawn after division of patients into a R group and a N-R group on the basis of metabolites after implementation of two cycles of treatment according to the equation (2).

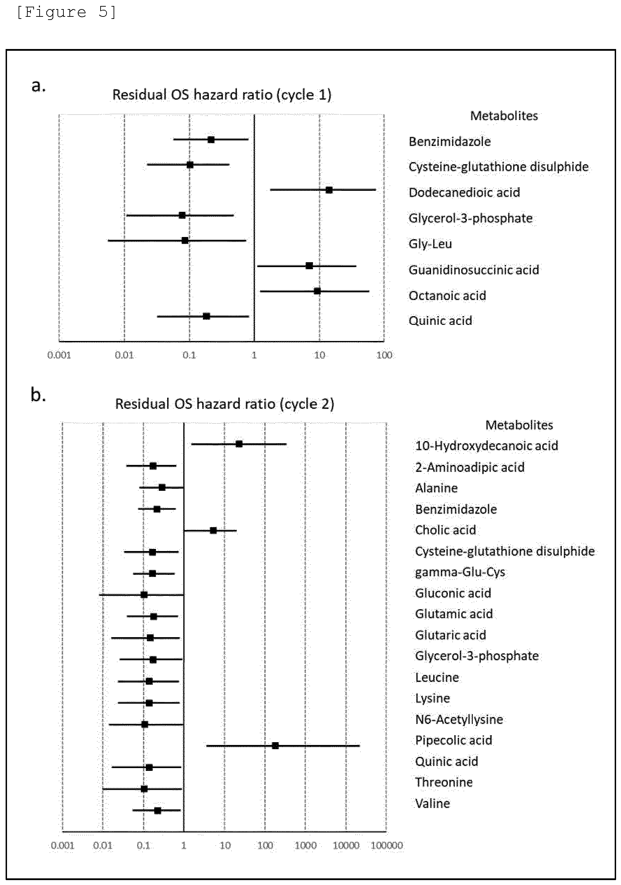

[Figure 5] Figure 5(a) is a diagram of the hazard ratios and 95% confidence intervals of metabolites which exhibited significant difference in analyzing the relationship between metabolite concentrations after implementation of one cycle of treatment and residual OS using COX proportional hazard model. Figure 5(b) is a diagram of the hazard ratios and 95% confidence intervals of metabolites which exhibited significant difference in analyzing the relationship between metabolite concentrations after implementation of two cycles of treatment and residual OS using COX proportional hazard model.

Modes for Carrying out the Invention

The marker for determining sensitivity to an anti-cancer agent early after the start of treatment with the anti-cancer agent according to the present invention includes 20 metabolite substances, i.e., 2-aminoadipic acid (2AMAD), 2-aminobutyric acid (2ABA), 2-cyanopyridine (2CYPR), 5-oxoproline (5OPRO), 6-aminohexanoic acid (6AHXA), adenosine (ADEN), aspartic acid (ASP), betonicine (BETNC), carbachol (CARB), cysteine-glutathione disulphide (CSSG), dopamine (DOPM), gamma-glutamylcysteine (GGLCY), oxidized glutathione (GSSG), hypotaurine (HYPT), methionine sulfoxide (METSF), N6-methyl-2'-deoxyadenosine (N6MDA), N-omega-methyltryptamine (NOMTR), phenyl phosphate (PHEP), proline (PRO) and ribulose 5-phosphate (RIB5P).

As shown in Examples mentioned later, the amounts of blood metabolites in blood specimens from colorectal cancer patients after implementation of one cycle of bevacizumab plus mFOLFOX6 therapy were comprehensively analyzed using CE-Q-TOF MS and CE-TOF MS. As a result, 2AMAD, 2CYPR, 6AHXA, BETNC, CSSG, GGLCY and GSSG were found to have a higher concentration in patients of a responder group having high therapeutic response to bevacizumab plus mFOLFOX6 therapy than in patients of a non-responder group having low therapeutic response thereto. CARB, N6MDA, NOMTR, PHEP and RIB5P were found to have a lower concentration in the patients of the responder group than in the patients of the non-responder group. Thus, these 12 substances are useful as markers for determining sensitivity to an anti-cancer agent comprising oxaliplatin or a salt thereof, fluorouracil or a salt thereof, and levofolinate or a salt thereof early after the start of treatment with the anti-cancer agent, particularly, markers for determining sensitivity to an anti-cancer agent comprising oxaliplatin or a salt thereof, fluorouracil or a salt thereof, levofolinate or a salt thereof, and bevacizumab early after the start of treatment with the anti-cancer agent. Among them, a combination of 5 substances, 2AMAD, BETNC, CSSG, N6MDA and RIB5P, is particularly useful, and use thereof permits determination with higher accuracy of whether or not a targeted cancer patient is a responder.

As shown in Examples mentioned later, the amounts of blood metabolites in blood specimens from colorectal cancer patients after implementation of two cycles of bevacizumab plus mFOLFOX6 therapy were comprehensively analyzed using CE-Q-TOF MS and CE-TOF MS. As a result, 2ABA, 5OPRO, 6AHXA, ADEN, DOPM, METSF and PRO were found to have a higher concentration in the patients of the responder group than in the patients of the non-responder group. ASP and HYPT were found to have a lower concentration in the patients of the responder group than in the patients of the non-responder group. Thus, these 9 substances are useful as markers for determining sensitivity to an anti-cancer agent comprising oxaliplatin or a salt thereof, fluorouracil or a salt thereof, and levofolinate or a salt thereof early after the start of treatment with the anti-cancer agent, particularly, markers for determining sensitivity to an anti-cancer agent comprising oxaliplatin or a salt thereof, fluorouracil or a salt thereof, levofolinate or a salt thereof, and bevacizumab early after the start of treatment with the anti-cancer agent. Among them, a combination of 6 substances, 2ABA, ASP, DOPM, HYPT, METSF and PRO, is particularly useful, and use thereof permits determination with higher accuracy of whether or not a targeted cancer patient is a responder.

The marker for predicting prognosis early after the start of treatment with an anti-cancer agent according to the present invention includes 22 metabolite substances, i.e., 10-hydroxydecanoic acid (10HDA), 2AMAD, alanine (ALA), benzimidazole (BEZIZ), cholic acid (CHOA), CSSG, dodecanedioic acid (DDNA), GGLCY, gluconic acid (GLCOA), glutamic acid (GLU), glutaric acid (GLTA), glycerol-3-phosphate (GLC3P), glycylleucine (GLYLE), guanidinosuccinic acid (GUSA), leucine (LEU), lysine (LYS), N6-acetyllysine (N6ALY), octanoic acid (OCTA), pipecolic acid (PIPEC), quinic acid (QUINA), threonine (THR) and valine (VAL).

As shown in Examples mentioned later, the amounts of blood metabolites in blood specimens from colorectal cancer patients after implementation of one cycle of bevacizumab plus mFOLFOX6 therapy and residual overall survivals (OS) were analyzed using COX proportional hazard model. As a result, it was found that the higher the blood concentrations of BEZIZ, CSSG, GLC3P, GLYLE and QUINA, the longer the survival period, and the higher the blood concentrations of DDNA, GUSA and OCTA, the shorter the survival period.

In addition, as shown in Examples mentioned later, the amounts of blood metabolites in blood specimens from colorectal cancer patients after implementation of two cycles of bevacizumab plus mFOLFOX6 therapy and residual overall survivals were analyzed using COX proportional hazard model. As a result, it was found that the higher the blood concentrations of 2AMAD, ALA, BEZIZ, CSSG, GGLCY, GLCOA, GLU, GLTA, GLC3P, LEU, LYS, N6ALY, QUINA, THR, the longer the survival period, and the higher the blood concentrations of 10HDA, CHOA and PIPEC, the shorter the survival period. Thus, each alone of these 22 substances is useful as a marker for predicting prognosis early after the start of treatment with an anti-cancer agent comprising oxaliplatin or a salt thereof, fluorouracil or a salt thereof, and levofolinate or a salt thereof, particularly, an anti-cancer agent comprising oxaliplatin or a salt thereof, fluorouracil or a salt thereof, levofolinate or a salt thereof, and bevacizumab, particularly, as a marker for predicting the length of OS, also including secondary treatment or subsequent treatment.

Herein, the "responder" refers to a patient who exhibits complete response or partial response in terms of the maximum effect during a study treatment period as a result of diagnostic imaging by radiologists according to the RECIST criteria (J Natl Cancer Inst. 2000 Feb 2; 92 (3): 205-16). The "non-responder" refers to a patient who exhibits stable disease or progressive disease in terms of the maximum effect during a study treatment period as a result of diagnostic imaging by radiologists according to the RECIST criteria.

In the present specification, the phrase "early after the start of treatment with an anti-cancer agent" refers to a state which has received at least one cycle, preferably one or more cycles and four or less cycles, more preferably one or more cycles and three or less cycles, further preferably one cycle or two cycles, of treatment with an anti-cancer agent comprising oxaliplatin or a salt thereof, fluorouracil or a salt thereof, and levofolinate or a salt thereof, particularly, an anti-cancer agent comprising oxaliplatin or a salt thereof, fluorouracil or a salt thereof, levofolinate or a salt thereof, and bevacizumab.

2AMAD is a metabolite in the lysine metabolic pathway. However, it is totally unknown that 2AMAD can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of one cycle of treatment.

Furthermore, it is totally unknown that 2AMAD can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

2ABA is a metabolite in the cysteine-methionine metabolic pathway. However, it is totally unknown that 2ABA can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of two cycles of treatment.

2CYPR is a pyridine derivative. However, it is totally unknown that 2CYPR can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of one cycle of treatment.

5OPRO is an amino acid which is formed from glutamic acid. However, it is totally unknown that 5OPRO can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of two cycles of treatment.

6AHXA, a caprolactam metabolite, is known to suppress fibrin degradation and thereby suppress bleeding through binding to plasminogen in a manner antagonistic to fibrin, and may be used as a hemostat. However, it is totally unknown that 6AHXA can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of one cycle or two cycles of treatment.

ADEN is used as a base in DNA or the like *in vivo.* However, it is totally unknown that ADEN can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of two cycles of treatment.

ASP, an amino acid, is generally known to play a central role in nitrogen processing *in vivo.* It has already been known that ASP can be used as a marker for determining sensitivity to an anti-cancer agent comprising oxaliplatin or a salt thereof and fluorouracil or a salt thereof, and has a higher concentration in a cell line with high sensitivity to oxaliplatin than in a cell line with low sensitivity thereto (WO 2013/125675). However, it is totally unknown that ASP can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of two cycles of treatment.

BETNC is a proline-related compound. However, it is totally unknown that BETNC can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of one cycle of treatment.

CARB is known as a substance stimulating parasympathetic nerve. However, it is totally unknown that CARB can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of one cycle of treatment.

Both CSSG and GSSG are metabolites of GSH known as a substance involved in the detoxification of drugs. CSSG is composed of GSH and Cys bound to each other. GSSG is a GSH dimer which is formed by the oxidation of GSH. The findings are known that: GSH in blood is rapidly oxidized into GSSG after blood collection; and more molecules of GSH bind rapidly after blood collection to Cys more abundant in blood than GSH to form CSSG in a few minutes, for example. There are no reports focused on drug efficacy and CSSG or GSSG, also including reports related to cancer. It is totally unknown that CSSG or GSSG can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of one cycle of treatment.

Furthermore, it is totally unknown that CSSG can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of one cycle or two cycles of treatment.

DOPM, a neurotransmitter present in the central nervous system, is a substance involved in motor regulation, hormonal regulation, emotion, willingness, learning, etc. However, it is totally unknown that DOPM can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of two cycles of treatment.

GGLCY is a metabolite in the glutathione metabolic pathway. However, it is totally unknown that GGLCY can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of one cycle of treatment.

Furthermore, it is totally unknown that GGLCY can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

HYPT, an intermediate product in the biosynthesis of taurine from cysteine, is synthesized by the oxidation of 3-sulfino-L-alanine or cysteamine. HYPT is known to have an anti-inflammatory effect (JP-A-2017-7980) and an antioxidative effect (JP-A-2017-14167). However, it is totally unknown that HYPT can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of two cycles of treatment.

METSF is known as a substance in the CYS and methionine metabolic pathways and also known as a biomarker for prostate cancer or oxidative stress or a biomarker for diagnosing depression. However, it is totally unknown that METSF can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of two cycles of treatment.

N6MDA is known as a nucleoside analog. However, it is totally unknown that N6MDA can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of one cycle of treatment.

NOMTR is known to be present in the barks, shoots, leaves, etc. of various types of plants. NOMTR, a receptor agonist of serotonin 4 receptor (5-HT4), is known to be detected from the urine of autism or epilepsy patients. However, it is totally unknown that NOMTR can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of one cycle of treatment.

PHEP is known as a product in the aminobenzoate metabolic pathway. However, it is totally unknown that PHEP can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of one cycle of treatment.

PRO is an amino acid. However, it is totally unknown that PRO can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its high concentration early after the start of treatment, particularly, after implementation of two cycles of treatment.

RIB5P is a final product of the pentose phosphate pathway. However, it is totally unknown that RIB5P can be used as a marker for determining sensitivity to an anti-cancer agent comprising oxaliplatin or a salt thereof, fluorouracil or a salt thereof, and levofolinate or a salt thereof early after the start of treatment with the anti-cancer agent, and permits determination of a responder by its low concentration early after the start of treatment, particularly, after implementation of one cycle of treatment.

10HDA is a compound which is contained in royal jelly. However, it is totally unknown that 10HDA can be used as a marker for predicting prognosis early after the start of treatment with an anti-cancer agent comprising oxaliplatin or a salt thereof, fluorouracil or a salt thereof, and levofolinate or a salt thereof, and permits determination of a shorter survival period by its higher concentration early after the start of treatment with the anti-cancer agent, particularly, after implementation of two cycles of treatment.

ALA, an amino acid, is known as a substance not only in the ALA, aspartate and glutamate metabolic pathways, the CYS and methionine metabolic pathways, and the taurine and hypotaurine metabolic pathways but in various metabolic pathways. ALA is also known as a biomarker for diagnosing depression or a prostate cancer biomarker. However, it is totally unknown that ALA can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

BEZIZ is a heterocyclic compound composed of a benzene ring and an imidazole ring. However, it is totally unknown that BEZIZ can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of one cycle or two cycles of treatment.

CHOA is a bile acid. However, it is totally unknown that CHOA can be used as a marker for predicting prognosis early after the start of treatment with an anti-cancer agent comprising oxaliplatin or a salt thereof, fluorouracil or a salt thereof, and levofolinate or a salt thereof, and permits determination of a shorter survival period by its higher concentration early after the start of treatment with the anti-cancer agent, particularly, after implementation of two cycles of treatment.

DDNA, a dicarboxylic acid, is known as an intermediate of fat and carbohydrate and also known as an index for hepatic carnitine palmitoyltransferase I deficiency. However, it is totally unknown that DDNA can be used as a marker for predicting prognosis early after the start of treatment with an anti-cancer agent comprising oxaliplatin or a salt thereof, fluorouracil or a salt thereof, and levofolinate or a salt thereof, and permits determination of a shorter survival period by its higher concentration early after the start of treatment with the anti-cancer agent, particularly, after implementation of one cycle of treatment.

GLCOA is a carboxylic acid which is formed by the oxidation of carbon at position 1 of glucose. However, it is totally unknown that GLCOA can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

GLU is an amino acid. However, it is totally unknown that GLU can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

GLTA is a substance involved in the citric acid cycle *in vivo*. However, it is totally unknown that GLTA can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

GLC3P is a metabolite in the glycerolipid or glycerophosphoric acid metabolic pathway or in the choline metabolic pathway in cancer. However, it is totally unknown that GLC3P can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of one cycle or two cycles of treatment.

GLYLE is an aminocarboxylic acid. However, it is totally unknown that GLYLE can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of one cycle of treatment.

GUSA is a compound considered as one of the causative substances of uremia. It has been pointed out that GUSA is involved in platelet dysfunction, particularly, inhibits the ability of platelet factor 3 and the platelet aggregation ability. However, it is totally unknown that GUSA can be used as a marker for predicting prognosis early after the start of treatment with an anti-cancer agent comprising oxaliplatin or a salt thereof, fluorouracil or a salt thereof, and levofolinate or a salt thereof, and permits determination of a shorter survival period by its higher concentration early after the start of treatment with the anti-cancer agent, particularly, after implementation of one cycle of treatment.

LEU, an essential amino acid, is known as a substance in the VAL, LEU and ILE synthetic and metabolic pathways. LEU is also known as a cancer biomarker in blood or a biomarker for diagnosing depression. However, it is totally unknown that LEU can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

LYS, an amino acid, is known as a substance in the LYS synthetic and metabolic pathways. LYS is also known as a saliva biomarker for cancer for oral cancer detection or a biomarker for diagnosing depression. However, it is totally unknown that LYS can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

N6ALY is known as a substance in the LYS metabolic pathway. However, it is totally unknown that N6ALY can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

OCTA is known as a metabolite in the fatty acid synthetic pathway. However, it is totally unknown that OCTA can be used as a marker for predicting prognosis early after the start of treatment with an anti-cancer agent comprising oxaliplatin or a salt thereof, fluorouracil or a salt thereof, and levofolinate or a salt thereof, and permits determination of a shorter survival period by its higher concentration early after the start of treatment with the anti-cancer agent, particularly, after implementation of one cycle of treatment.

PIPEC is a substance in the lysine metabolic pathway and is formed with lysine as a starting material *in vivo.* Its plasma concentration is known to be elevated during chronic liver damage. However, it is totally unknown that PIPEC can be used as a marker for predicting prognosis early after the start of treatment with an anti-cancer agent comprising oxaliplatin or a salt thereof, fluorouracil or a salt thereof, and levofolinate or a salt thereof, and permits determination of a shorter survival period by its higher concentration early after the start of treatment with the anti-cancer agent, particularly, after implementation of two cycles of treatment.

QUINA is a metabolite in the phenylalanine-tyrosine-tryptophan synthetic pathway. However, it is totally unknown that QUINA can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of one cycle or two cycles of treatment.

THR, an amino acid, is known as a substance in the glycine, SER and THR metabolic pathways and as a substance in the VAL, LEU and ILE synthetic and metabolic pathways. THR has already been known as a biomarker for diagnosing depression. However, it is totally unknown that THR can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

VAL, an essential amino acid, is known as a substance in the VAL, LEU and ILE synthetic and metabolic pathways. VAL is also known as a cancer biomarker in blood or a biomarker for diagnosing depression. However, it is totally unknown that VAL can be used as a marker for predicting prognosis early after the start of treatment with an anti-cancer agent comprising oxaliplatin or a salt thereof, fluorouracil or a salt thereof, and levofolinate or a salt thereof, and permits determination of a longer survival period by its higher concentration early after the start of treatment with the anti-cancer agent, particularly, after implementation of two cycles of treatment.

The anti-cancer agent targeted by the marker for determining sensitivity to an anti-cancer agent early after the start of treatment with the anti-cancer agent according to the present invention is an anti-cancer agent comprising oxaliplatin or a salt thereof, fluorouracil or a salt thereof, and levofolinate or a salt thereof. An anti-cancer agent which is converted to oxaliplatin, fluorouracil or levofolinate through metabolism *in vivo* is also targeted by the marker of the present invention. Specifically, it has been revealed that tegafur or capecitabine is converted to fluorouracil through metabolism *in vivo*. Therefore, an anti-cancer agent comprising oxaliplatin or a salt thereof, tegafur or a salt thereof, and levofolinate or a salt thereof, or an anti-cancer agent comprising oxaliplatin or a salt thereof, capecitabine or a salt thereof, and levofolinate or a salt thereof is also targeted by the marker of the present invention.

Examples of an additional anti-cancer agent for use in combination with the anti-cancer agent comprising oxaliplatin or a salt thereof, fluorouracil or a salt thereof, and levofolinate or a salt thereof include, but are not particularly limited to, cyclophosphamide, ifosfamide, thiotepa, melphalan, busulfan, nimustine, ranimustine, dacarbazine, procarbazine, temozolomide, cisplatin, carboplatin, nedaplatin, methotrexate, pemetrexed, tegafur/uracil, doxifluridine, tegafur/gimeracil/oteracil, capecitabine, cytarabine, enocitabine, gemcitabine, 6-mercaptopurine, fludarabine, pentostatin, cladribine, hydroxyurea, doxorubicin, epirubicin, daunorubicin, idarubicine, pirarubicin, mitoxantrone, amrubicin, actinomycin D, bleomycine, pepleomycin, mitomycin C, aclarubicin, zinostatin, vincristine, vindesine, vinblastine, vinorelbine, paclitaxel, docetaxel, irinotecan, anirinotecan active metabolite SN-38, nogitecan, topotecan, etoposide, prednisolone, dexamethasone, tamoxifen, toremifene, medroxyprogesterone, anastrozole, exemestane, letrozole, rituximab, imatinib, gefitinib, gemtuzumab ozogamicin, bortezomib, erlotinib, cetuximab, bevacizumab, sunitinib, sorafenib, dasatinib, panitumumab, asparaginase, tretinoin, arsenic trioxide, and salts thereof, and active metabolites thereof. Among them, irinotecan or SN-38, or a salt thereof, or bevacizumab is preferred, and bevacizumab is particularly preferred.

The determination of sensitivity to an anti-cancer agent using the marker for determining sensitivity to an anti-cancer agent early after the start of treatment with the anti-cancer agent according to the present invention can be performed by measuring an amount of one or more molecules selected from the group consisting of 2AMAD, 2ABA, 2CYPR, 5OPRO, 6AHXA, ADEN, ASP, BETNC, CARB, CSSG, DOPM, GGLCY, GSSG, HYPT, METSF, N6MDA, NOMTR, PHEP, PRO and RIB5P in a biological sample (specimen) derived from a cancer patient early after the start of treatment with an anti-cancer agent comprising oxaliplatin or a salt thereof, fluorouracil or a salt thereof, and levofolinate or a salt thereof, and specifically, further comparing the measurement result with a control level (a standard concentration, a concentration range in a responder, a cutoff value of a responder (hereinafter, the cutoff value means a relative concentration when the concentration of an internal standard solution for LC/MS is defined as 1), etc.).

Specifically, the determination of sensitivity to an anti-cancer agent using one or more molecules selected from the group consisting of 2AMAD, 2CYPR, 6AHXA, BETNC, CARB, CSSG, GGLCY, GSSG, N6MDA, NOMTR, PHEP and RIB5P as the marker for determining sensitivity to an anti-cancer agent early after the start of treatment with the anti-cancer agent according to the present invention can be performed by measuring an amount of the one or more molecules in a biological sample (specimen) derived from a cancer patient after implementation of one cycle of treatment with an anti-cancer agent comprising oxaliplatin or a salt thereof, fluorouracil or a salt thereof, and levofolinate or a salt thereof, and specifically, further comparing the measurement result with a control level (a standard concentration, a concentration range in a responder, a cutoff value of a responder, etc.). Alternatively, this determination can be performed by measuring amounts of 2AMAD, BETNC, CSSG, N6MDA and RIB5P in a biological sample (specimen) derived from a cancer patient after implementation of one cycle of treatment with the anti-cancer agent, and specifically, further digitizing the measurement results by comparison with a cutoff value of a responder of each substance, and assigning the obtained numeric values into a particular calculation expression.

The determination of sensitivity to an anti-cancer agent using one or more molecules selected from the group consisting of 2ABA, 5OPRO, 6AHXA, ADEN, ASP, DOPM, HYPT, METSF and PRO as the marker for determining sensitivity to an anti-cancer agent early after the start of treatment with the anti-cancer agent according to the present invention can be performed by measuring an amount of the one or more molecules in a biological sample (specimen) derived from a cancer patient after implementation of two cycles of treatment with an anti-cancer agent comprising oxaliplatin or a salt thereof, fluorouracil or a salt thereof, and levofolinate or a salt thereof, and specifically, further comparing the measurement result with a control level (a standard concentration, a concentration range in a responder, a cutoff value of a responder, etc.). Alternatively, this determination can be performed by measuring amounts of 2ABA, ASP, DOPM, HYPT, METSF and PRO in a biological sample (specimen) derived from a cancer patient after implementation of two cycles of treatment with the anti-cancer agent, and specifically, further digitizing the measurement results by comparison with a cutoff value of a responder of each substance, and assigning the obtained numeric values into a particular calculation expression.

A method for predicting long-term prognosis for treatment with an anti-cancer agent using the marker for predicting prognosis early after the start of treatment with the anti-cancer agent according to present invention can be performed by measuring an amount of one or more molecules selected from the group consisting of 10HDA, 2AMAD, ALA, BEZIZ, CHOA, CSSG, DDNA, GGLCY, GLCOA, GLU, GLTA, GLC3P, GLYLE, GUSA, LEU, LYS, N6ALY, OCTA, PIPEC, QUINA, THR and VAL in a biological sample (specimen) derived from a cancer patient early after the start of treatment with an anti-cancer agent comprising oxaliplatin or a salt thereof, fluorouracil or a salt thereof, and levofolinate or a salt thereof, and specifically, further comparing the measurement result with a control level (a standard concentration, a cutoff value of a responder, etc.).

Specifically, a method for predicting prognosis using one or more molecules selected from the group consisting of BEZIZ, CSSG, DDNA, GLC3P, GLYLE, GUSA, OCTA and QUINA as the marker for predicting prognosis early after the start of treatment with an anti-cancer agent according to the present invention can be performed by measuring an amount of the one or more molecules in a biological sample (specimen) derived from a cancer patient after implementation of one cycle of treatment with an anti-cancer agent comprising oxaliplatin or a salt thereof, fluorouracil or a salt thereof, and levofolinate or a salt thereof, and specifically, further comparing the measurement result with a control level (a standard concentration, a cutoff value of a responder, etc.).

A method for predicting prognosis using one or more molecules selected from the group consisting of 10HDA, 2AMAD, ALA, BEZIZ, CHOA, CSSG, GGLCY, GLCOA, GLU, GLTA, GLC3P, LEU, LYS, N6ALY, PIPEC, QUINA, THR and VAL as the marker for predicting prognosis early after the start of treatment with an anti-cancer agent according to the present invention can be performed by measuring an amount of the one or more molecules in a biological sample (specimen) derived from a cancer patient after implementation of two cycles of treatment with an anti-cancer agent comprising oxaliplatin or a salt thereof, fluorouracil or a salt thereof, and levofolinate or a salt thereof, and specifically, further comparing the measurement result with a control level (a standard concentration, a cutoff value of a responder, etc.).

In this context, the cancer patient encompasses a test subject having cancer or a test subject who had cancer. Examples of the biological sample include blood, serum, plasma, a cancer tissue biopsy specimen, a cancer extirpation specimen, feces, urine, ascitic fluid, pleural effusion, cerebrospinal fluid, and sputum. Serum is particularly preferred.

Examples of the targeted cancer of the present invention include lip, oral and pharyngeal cancers typified by pharyngeal cancer; gastrointestinal tract cancers typified by esophageal cancer, gastric cancer, colorectal cancer, etc.; respiratory and pleural organ cancers typified by lung cancer, bone and articular cartilage cancers; malignant melanoma of the skin, squamous cell cancer and other cancers of the skin; mesothelial and soft tissue cancers typified by mesothelioma; female genital cancers typified by breast cancer, uterine cancer, and ovarian cancer; male genital cancers typified by prostate cancer; urinary tract cancers typified by bladder cancer; eye, brain and central nervous system cancers typified by brain tumor; thyroid and other endocrine cancers; lymphoid tissue, hematopoietic tissue and related tissue cancers typified by non-Hodgkin's lymphoma and lymphoid leukemia; and metastatic cancers from the aforementioned cancers as primary foci. Colorectal cancer (large intestine cancer) can be suitable as a target, and chemotherapeutically untreated cancer is particularly preferred.

The means for measuring the molecule selected from the group consisting of 2AMAD, 2ABA, 2CYPR, 5OPRO, 6AHXA, ADEN, ASP, BETNC, CARB, CSSG, DOPM, GGLCY, GSSG, HYPT, METSF, N6MDA, NOMTR, PHEP, PRO, RIB5P, 10HDA, ALA, BEZIZ, CHOA, DDNA, GLCOA, GLU, GLTA, GLC3P, GLYLE, GUSA, LEU, LYS, N6ALY, OCTA, PIPEC, QUINA, THR and VAL in a specimen may be appropriately determined according to the substance to be measured, and may be measured by use of, for example, various mass spectrometers such as CE-Q-TOF MS, CE-TOF MS, or gas chromatography-mass spectrometry (GC-MS), HPLC, an immunological measurement method, or a biochemical assay.

In order to determine sensitivity to the targeted anti-cancer agent using one or more molecules selected from the group consisting of 2AMAD, 2CYPR, 6AHXA, BETNC, CSSG, GGLCY and GSSG, the amount, for example, concentration, of one or more molecules selected from the group consisting of 2AMAD, 2CYPR, 6AHXA, BETNC, CSSG, GGLCY and GSSG in a biological sample derived from a cancer patient may be measured after implementation of one cycle of treatment with the anti-cancer agent. When the concentration has a concentration confirmed to be higher than the predetermined control level, the cancer can be determined to be sensitive to the targeted anti-cancer agent. Therefore, these markers for determining sensitivity to an anti-cancer agent early after the start of treatment with the anti-cancer agent can be used as markers for aggressively continuing the treatment of patients who can be expected to receive therapeutic effects. On the other hand, when the concentration has a concentration confirmed to be lower than the predetermined control level, the cancer can be determined to be not sensitive to the targeted anti-cancer agent. If the cancer has no sensitivity to the targeted anti-cancer agent, the administration of such an anti-cancer agent which cannot be expected to produce therapeutic effects is continued. Thus, there is a fear on the progression of cancer or adverse reactions. Thus, the marker for determining sensitivity to an anti-cancer agent early after the start of treatment with the anti-cancer agent according to the present invention can be used as a marker for aggressively continuing the treatment of patients who can be expected to receive therapeutic effects, and in addition, can also be used as a marker for circumventing the progression of cancer and adverse reactions associated with the continuous administration of an anti-cancer agent which cannot be expected to produce therapeutic effects.

Examples of the control level include a cutoff value. Examples of the cutoff value include $2.091 \times 10^{-2} \leq$ for 2AMAD, $3.444 \times 10^{-3} \leq$ for 2CYPR, $7.175 \times 10^{-3} \leq$ for 6AHXA, $1.589 \times 10^{-2} \leq$ for BETNC, $2.198 \times 10^{-2} \leq$ for CSSG,

$7.389 \times 10^{-3} \leq$ for GGLCY, and $5.606 \times 10^{-4} \leq$ for GSSG.

In order to determine sensitivity to the targeted anti-cancer agent using one or more molecules selected from the group consisting of CARB, N6MDA, NOMTR, PHEP and RIB5P, the amount, for example, concentration, of one or more molecules selected from the group consisting of CARB, N6MDA, NOMTR, PHEP and RIB5P in a biological sample derived from a cancer patient may be measured after implementation of one cycle of treatment with the anti-cancer agent. When the concentration has a concentration confirmed to be lower than the predetermined control level, the cancer can be determined to be sensitive to the targeted anti-cancer agent. Therefore, these markers for determining sensitivity to an anti-cancer agent early after the start of treatment with the anti-cancer agent can be used as markers for aggressively continuing the treatment of patients who can be expected to receive therapeutic effects. On the other hand, when the concentration has a concentration confirmed to be higher than the predetermined control level, the cancer can be determined to be not sensitive to the targeted anti-cancer agent. If the cancer has no sensitivity to the targeted anti-cancer agent, the administration of such an anti-cancer agent which cannot be expected to produce therapeutic effects is continued. Thus, there is a fear on the progression of cancer or adverse reactions. Thus, the marker for determining sensitivity to an anti-cancer agent early after the start of treatment with the anti-cancer agent according to the present invention can be used as a marker for aggressively continuing the treatment of patients who can be expected to receive therapeutic effects, and in addition, can also be used as a marker for circumventing the progression of cancer and adverse reactions associated with the continuous administration of an anti-cancer agent which cannot be expected to produce therapeutic effects.

Examples of the control level include a cutoff value. Examples of the cutoff value include $\leq 8.472 \times 10^{-3}$ for CARB, $\leq 1.208 \times 10^{-3}$ for N6MDA, $\leq 7.006 \times 10^{-2}$ for NOMTR, $\leq 1.801 \times 10^{-2}$ for PHEP, and $\leq 3.005 \times 10^{-3}$ for RIB5P.

In order to determine sensitivity to the targeted anti-cancer agent using 2AMAD, BETNC, CSSG, N6MDA and RIB5P, the amounts, for example, concentrations, of 2AMAD, BETNC, CSSG, N6MDA and RIB5P in a biological sample derived from a cancer patient may be measured after implementation of one cycle of treatment with the anti-cancer agent, and the predetermined numeric values according to the measurement results may be assigned to the equation (1):

$$p = \frac{1}{1 + e^{(-16.4681 + (2AMAD) + (BETNC) + (CSSG) + (N6MDA) + (RIB5P))}} \qquad (1)$$

wherein 2AMAD represents -16.2688 when a measurement result about 2AMAD is equal to or more than a cutoff value, and 16.2688 when the measurement result is less than the cutoff value; BETNC represents -8.6560 when a measurement result about BETNC is equal to or more than a cutoff value, and 8.6560 when the measurement result is less than the cutoff value; CSSG represents -1.4372 when a measurement result about CSSG is equal to or more than a cutoff value, and 1.4372 when the measurement result is less than the cutoff value; N6MDA represents -8.6658 when a measurement result about N6MDA is equal to or less than a cutoff value, and 8.6658 when the measurement result is more than the cutoff value; and RIB5P represents -1.3451 when a measurement result about RIB5P is equal to or less than a cutoff value, and 1.3451 when the measurement result is more than the cutoff value.

The cutoff value of each substance is $2.091 \times 10^{-2}$ for 2AMAD, $1.589 \times 10^{-2}$ for BETNC, $2.198 \times 10^{-2}$ for CSSG, $1.208 \times 10^{-3}$ for N6MDA, and $3.005 \times 10^{-3}$ for RIB5P.

p calculated according to the equation (1) represents a probability that the targeted cancer patient is a responder. When p is more than 0.5, cancer in the cancer patient can be determined to be sensitive to the targeted anti-cancer agent, i.e., the cancer patient can be determined to be a responder. Therefore, these markers for determining sensitivity to an anti-cancer agent early after the start of treatment with the anti-cancer agent can be used as markers for aggressively continuing the treatment of patients who can be expected to receive therapeutic effects. On the other hand, when p is 0.5 or less, cancer in the cancer patient can be determined to be not sensitive to the targeted anti-cancer agent, i.e., the cancer patient can be determined to be a non-responder. If the cancer has no sensitivity to the targeted anti-cancer agent, the administration of such an anti-cancer agent which cannot be expected to produce therapeutic effects is continued. Thus, there is a fear on the progression of cancer or adverse reactions. Thus, the marker for determining sensitivity to an anti-cancer agent early after the start of treatment with the anti-cancer agent according to the present invention can be used as a marker for aggressively continuing the treatment of patients who can be expected to receive therapeutic effects, and in addition, can also be used as a marker for circumventing the progression of cancer and adverse reactions associated with the continuous administration of an anti-cancer agent which cannot be expected to have drug efficacy.

In order to determine sensitivity to the targeted anti-cancer agent using one or more molecules selected from the group consisting of 2ABA, 5OPRO, 6AHXA, ADEN, DOPM, METSF and PRO, the amount, for example, concentration, of one or more molecules selected from the group consisting of 2ABA, 5OPRO, 6AHXA, ADEN, DOPM, METSF and PRO in a biological sample derived from a cancer patient may be measured after implementation of two cycles of treatment with the anti-cancer agent. When the concentration has a concentration confirmed to be higher than the predetermined control level, the cancer can be determined to be sensitive to the targeted anti-cancer agent. Therefore, these markers for determining sensitivity to an anti-cancer agent early after the start of treatment with the anti-cancer agent can be used as markers for aggressively continuing the treatment of patients who can be expected to receive therapeutic effects. On the other hand, when the concentration has a concentration confirmed to be lower than the predetermined control level, the cancer can be determined to be not sensitive to the targeted anti-cancer agent. If the cancer has no sensitivity to the targeted anti-cancer agent, the administration of such an anti-cancer agent which cannot be expected to produce therapeutic effects is continued. Thus, there is a fear on the progression of cancer or adverse reactions. Thus, the marker for determining sensitivity to an anti-cancer agent early after the start of treatment with the anti-cancer agent according to the present invention can be used as a marker for aggressively continuing the treatment of patients who can be expected to receive therapeutic effects, and in addition, can also be used as a marker for circumventing the progression of cancer and adverse reactions associated with the continuous administration of an anti-cancer agent which cannot be expected to have drug efficacy.

Examples of the control level include a cutoff value. Examples of the cutoff value include $2.507 \times 10^{-1} \leq$ for 2ABA, $1.843 \times 10^{-1} \leq$ for 50PRO, $4.568 \times 10^{-3} \leq$ for 6AHXA, $2.305 \times 10^{-2} \leq$ for ADEN, $5.606 \times 10^{-4} \leq$ for DOPM, $3.836 \times 10^{-2} \leq$ for METSF, and $2.9876 \leq$ for PRO.

In order to determine sensitivity to the targeted anti-cancer agent using one or more molecules selected from the group consisting of ASP and HYPT, the amount, for example, concentration, of one or more molecules selected from the group consisting of ASP and HYPT in a biological sample derived from a cancer patient may be measured after implementation of two cycles of treatment with the anti-cancer agent. When the concentration has a concentration confirmed to be lower than the predetermined control level, the cancer can be determined to be sensitive to the targeted anti-cancer agent. Therefore, these markers for determining sensitivity to an anti-cancer agent early after the start of treatment with the anti-cancer agent can be used as markers for aggressively continuing the treatment of patients who can be expected to receive therapeutic effects. On the other hand, when the concentration has a concentration confirmed to be higher than the predetermined control level, the cancer can be determined to be not sensitive to the targeted anti-cancer agent. If the cancer has no sensitivity to the targeted anti-cancer agent, the administration of such an anti-cancer agent which cannot be expected to produce therapeutic effects is continued. Thus, there is a fear on the progression of cancer or adverse reactions. Thus, the marker for determining sensitivity to an anti-cancer agent early after the start of treatment with the anti-cancer agent according to the present invention can be used as a marker for aggressively continuing the treatment of patients who can be expected to receive therapeutic effects, and in addition, can also be used as a marker for circumventing the progression of cancer and adverse reactions associated with the continuous administration of an anti-cancer agent which cannot be expected to have drug efficacy.

Examples of the control level include a cutoff value. Examples of the cutoff value include $\leq 1.170 \times 10^{-1}$ for ASP and $\leq 1.768 \times 10^{-2}$ for HYPT.

In order to determine sensitivity to the targeted anti-cancer agent using 2ABA, ASP, DOPM, HYPT, METSF and PRO, the amounts, for example, concentrations, of 2ABA, ASP, DOPM, HYPT, METSF and PRO in a biological sample derived from a cancer patient may be measured after implementation of two cycles of treatment with the anti-cancer agent, and the predetermined numeric values according to the measurement results may be assigned to the equation (2):

$$p = \frac{1}{1 + e^{(6.4305 + (2ABA) + (ASP) + (DOPM) + (HYPT) + (METSF) + (PRO))}} \qquad (2)$$

wherein 2ABA represents -2.3059 when a measurement result about 2ABA is equal to or more than a cutoff value, and 2.3059 when the measurement result is less than the cutoff value; ASP represents -1.8154 when a measurement result about ASP is equal to or less than a cutoff value, and 1.8154 when the measurement result is more than the cutoff value; DOPM represents -1.6345 when a measurement result about DOPM is equal to or more than a cutoff value, and 1.6345 when the measurement result is less than the cutoff value; HYPT represents -2.4113 when a measurement result about HYPT is equal to or less than a cutoff value, and 2.4113 when the measurement result is more than the cutoff value; METSF represents -1.5555 when a measurement result about METSF is equal to or more than a cutoff value, and 1.5555 when the measurement result is less than the cutoff value; and PRO represents -9.0794 when a measurement result about PRO is equal to or more than a cutoff value, and 9.0794 when the measurement result is less than the cutoff value.

The cutoff value of each substance is $2.507 \times 10^{-1}$ for 2ABA, $1.170 \times 10^{-1}$ for ASP, $5.606 \times 10^{-4}$ for DOPM, $1.768 \times 10^{-2}$ for HYPT, $3.836 \times 10^{-2}$ for METSF, and $2.9876$ for PRO.

p calculated according to the equation (2) represents a probability that the targeted cancer patient is a responder. When p is more than 0.5, cancer in the cancer patient can be determined to be sensitive to the targeted anti-cancer agent, i.e., the cancer patient can be determined to be a responder. Therefore, these markers for determining sensitivity to an anti-cancer agent early after the start of treatment with the anti-cancer agent can be used as markers for aggressively continuing the treatment of patients who can be expected to receive therapeutic effects. On the other hand, when p is 0.5 or less, cancer in the cancer patient can be determined to be not sensitive to the targeted anti-cancer agent, i.e., the cancer patient can be determined to be a non-responder. If the cancer has no sensitivity to the targeted anti-cancer agent, the administration of such an anti-cancer agent which cannot be expected to produce therapeutic effects is continued. Thus, there is a fear on the progression of cancer or adverse reactions. Thus, the marker for determining sensitivity to an anti-cancer agent early after the start of treatment with the anti-cancer agent according to the present invention can be used as a marker for aggressively continuing the treatment of patients who can be expected to receive therapeutic effects, and in addition, can also be used as a marker for circumventing the progression of cancer and adverse reactions associated with the continuous administration of an anti-cancer agent which cannot be expected to have drug efficacy.

In order to predict prognosis for treatment with the targeted anti-cancer agent using one or more molecules selected from the group consisting of BEZIZ, CSSG, GLC3P, GLYLE and QUINA, the amount, for example, concentration, of one or more molecules selected from the group consisting of BEZIZ, CSSG, GLC3P, GLYLE and QUINA in a biological sample derived from a cancer patient may be measured after implementation of one cycle of treatment with the anti-cancer agent. When the concentration has a concentration confirmed to be higher than the predetermined control level, good prognosis can be predicted as compared with when the concentration has a concentration confirmed to be lower than the predetermined control level.

In order to predict prognosis for treatment with the targeted anti-cancer agent using one or more molecules selected from the group consisting of DDNA, GUSA and OCTA, the amount, for example, concentration, of one or more molecules selected from the group consisting of DDNA, GUSA and OCTA in a biological sample derived from a cancer patient may be measured after implementation of one cycle of treatment with the anti-cancer agent. When the concentration has a concentration confirmed to be lower than the predetermined control level, good prognosis can be predicted as compared with when the concentration has a concentration confirmed to be higher than the predetermined control level.

In order to predict prognosis for treatment with the targeted anti-cancer agent using one or more molecules selected from the group consisting of 2AMAD, ALA, BEZIZ, CSSG, GGLCY, GLCOA, GLU, GLTA, GLC3P, LEU, LYS, N6ALY, QUINA, THR and VAL, the amount, for example, concentration, of one or more molecules selected from the group consisting of 2AMAD, ALA, BEZIZ, CSSG, GGLCY, GLCOA, GLU, GLTA, GLC3P, LEU, LYS, N6ALY, QUINA, THR and VAL in a biological sample derived from a cancer patient may be measured after implementation of two cycles of treatment with the anti-cancer agent. When the concentration has a concentration confirmed to be higher than the predetermined control level, good prognosis can be predicted as compared with when the concentration has a concentration confirmed to be lower than the predetermined control level.

In order to predict prognosis for treatment with the targeted anti-cancer agent using one or more molecules selected from the group consisting of 10HDA, CHOA and PIPEC, the amount, for example, concentration, of one or more molecules selected from the group consisting of 10HDA, CHOA and PIPEC in a biological sample derived from a cancer patient may be measured after implementation of two cycles of treatment with the anti-cancer agent. When the concentration has a concentration confirmed to be lower than the predetermined control level, good prognosis can be predicted as compared with when the concentration has a concentration confirmed to be higher than the predetermined control level.

For carrying out the method for determining sensitivity to an anti-cancer agent early after the start of treatment with the anti-cancer agent according to the present invention, it is preferred to use a kit comprising a protocol for measuring one or more molecules selected from the group consisting of 2AMAD, 2ABA, 2CYPR, 50PRO, 6AHXA, ADEN, ASP, BETNC, CARB, CSSG, DOPM, GGLCY, GSSG, HYPT, METSF, N6MDA, NOMTR, PHEP, PRO and RIB5P in a specimen. For carrying out the method for predicting prognosis early after the start of treatment with an anti-cancer agent according to the present invention, it is preferred to use a kit comprising a protocol for measuring one or more molecules selected from the group consisting of 10HDA, 2AMAD, ALA, BEZIZ, CHOA, CSSG, DDNA, GGLCY, GLCOA, GLU, GLTA, GLC3P, GLYLE, GUSA, LEU, LYS, N6ALY, OCTA, PIPEC, QUINA, THR and VAL in a specimen. The kit comprises a reagent for measuring these metabolite substances, and a protocol (a method for using the measurement reagent, and criteria for determining the presence or absence of anti-cancer agent sensitivity, etc.). The criteria include standard concentrations of these metabolite substances, their concentrations which are confirmed to be high, their concentrations which are confirmed to be low, factors which influence measurement results, the degree of the influence, etc. These concentrations can be set for each targeted anti-cancer agent. Determination or prediction can be performed as described above using the criteria.

Examples

Next, the present invention will be described in more detail with reference to Examples. However, the present invention is not limited by these examples by any means.

(1) Method

(a) Reagent

Methanol for LC/MS (manufactured by Wako Pure Chemical Industries, Ltd.), chloroform for HPLC (manufactured by Wako Pure Chemical Industries, Ltd.), and reverse osmosis water (Direct-Q UV, manufactured by Merck Millipore) were used in dissolution and sample preparation.

The internal standard solutions for LC/MS (cation) used were Internal Standard Solution Compound C1 (ISC1) and Internal Standard Solution Compound C2 (ISC2). ISC1 contains 10 mM L-methionine sulfone (manufactured by Human Metabolome Technologies America Inc.) in an aqueous solution. ISC2 contains 10 mM L-arginine-$^{13}C_6$ hydrochloride (manufactured by Sigma-Aldrich Co. LLC), L-asparagine-$^{15}N_2$ monohydrate (manufactured by Cambridge Isotope Laboratories, Inc.), β-alanine-$^{13}C_3$,$^{15}N$ (manufactured by Sigma-Aldrich Co. LLC), and tubercidin (manufactured by Sigma-Aldrich Co. LLC) in an aqueous solution.

The internal standard solutions for LC/MS (anion) used were Internal Standard Solution Compound A1 (ISA1) and Internal Standard Solution compound A2 (ISA2). ISA1 contains 10 mM D-camphor-10-sulfonic acid sodium salt (manufactured by Human Metabolome Technologies America Inc.) in an aqueous solution. ISA2 contains 10 mM chloranilic acid (manufactured by Tokyo Chemical Industry Co., Ltd.) in an aqueous solution.

These internal standard solutions were used for standardizing signal intensity and adjusting a migration time. Also, ISC1 and ISA1 were used for calculating the relative concentration of each metabolite.

(b) Clinical sample

(b-1) Patient background

Serum samples were prospectively collected from a total of 68 patients with histologically confirmed advanced colorectal cancer (ACRC) and eligible as subjects for primary treatment of standard chemotherapy. Criteria for registration (eligibility) in this trial are as follows.

- Age at the time of registration is equal to or over 20 years.
- Performance status (PS) of Eastern Corporative Oncology Group (ECOG) is 0 or 1.
- Histopathologically confirmed to have colorectal cancer
- Incurable and unresectable chemotherapeutically untreated advanced or recurrent disease (except for cases with postoperative adjuvant chemotherapy with 5-FU drugs which were registrable as long as the therapy had been completed up to 6 months before the date at which recurrence was confirmed)
- Having a predicted survival period of 3 months or longer
- Having no severe dysfunction in major organs
- A written informed consent with patient's own signature and date was obtained as to enrollment in tests including a gene polymorphism test and proteome or metabolome analysis before registration in this trial.

(b-2) Treatment of patient

All the patients underwent primary chemotherapy involving intravenous administration of bevacizumab (BV) at 5 mg/kg over from 30 to 90 minutes and subsequent intravenous administration of oxaliplatin (L-OHP) at 85 mg/m² and levofolinate (1-LV) at 200 mg/m² over 120 minutes. Then, 5-FU was intravenously bolus-administered at 400 mg/m² and subsequently, intravenously administered at 2,400 mg/m² by continuous infusion over 46 hours (mFOLFOX6 therapy). This treatment was repeated every 2 weeks.

After discontinuation of the treatment with L-OHP, simplified concomitant administration of 1-LV and 5-FU (sLV5FU2) was also confirmed as study treatment, if necessary, regardless of the presence or absence of BV treatment.

Up to 24 cycles were continued as study treatment unless there was disease progression, appearance of an adverse event due to which further study treatment must be discontinued, doctor's judgment, patient's rejection of continuation of study treatment, shift to curative or resectional surgery of tumor, etc.

(b-3) Evaluation of anti-tumor effect

The anti-tumor effects were evaluated on the basis of the Response Evaluation Criteria in Solid Tumors Guideline 1.0 (RECIST) by the independent external review board.

A total tumor size before treatment was measured using an image taken with a computerized topography or a magnetic resonance imaging within 1 month before registration. A total tumor size after the start of treatment was measured using images repetitively taken every 8 weeks in the same way as that before treatment.

(b-4) Collection of sample

A blood sample was collected within 2 weeks before the start of chemotherapy and 2 weeks after implementation of chemotherapy of each treatment cycle from the start of chemotherapy to the discontinuation of treatment with oxaliplatin.
The collected blood specimen was left at room temperature for 15 minutes for coagulation and then centrifuged at 3,000 rpm at 4°C for 30 minutes. Then, serum was transferred in equal amounts to four polypropylene tubes and immediately frozen with liquid nitrogen. All of these procedures were completed within 1 hour from blood collection. The serum sample was stored at -80°C until analysis.

(b-5) Preparation of sample

Sample preparation was performed in accordance with the previously reported method (J Proteome Res. 2003 Sep-Oct; 2 (5): 488-94; Metabolomics. 2010 Mar; 6 (1): 78-95; and Metabolomics. 2013 Apr ;9 (2): 444-453). The serum sample was thawed on ice, and 200 $\mu$L of the serum, the internal standard (10 $\mu$M ISA1 or ISC1), 2,000 $\mu$L of chloroform and 800 $\mu$L of reverse osmosis water were placed in a centrifugal tube containing 1,800 $\mu$L of methanol and mixed therewith. After vortexing, the mixture was centrifuged at 4,600 g at 4°C for 5 minutes. Then, 1,500 $\mu$L of the upper layer was transferred to a 5 kDa filter (manufactured by Merck Millipore) for the removal of proteins and centrifugally filtered at 9,100 g at 4°C for 2 to 4 hours. The filtrate was dried in a vacuum centrifuge. Immediately before CE-TOF MS analysis, the dried filtrate was dissolved in 50 $\mu$L of reverse osmosis water containing ISC2 or ISA2 (final concentration: 0.1 mM) on ice, placed in an analysis vial, centrifuged at 1,000 g at 4°C for 10 minutes, and subjected to analysis.

(c) Measurement of metabolite substance in sample by CE-TOF MS

All the samples were measured in duplicate. Metabolite substances having a mass number of 1,000 or smaller were comprehensively measured under cation measurement conditions using CE-Q-TOF MS or under anion measurement conditions using CE-TOF MS.

(c-1) Cationic metabolite substance measurement conditions

1) Measurement equipment

Agilent 6530 Accurate-Mass Q-TOF MS system equipped with Agilent 7100 CE system (manufactured by Agilent Technologies, Inc.) was used in the measurement of cationic metabolite substances. The capillary used was a fused silica capillary (inside diameter: 50 $\mu$m, total length: 80 cm) of catalogue number (Cat. No.) H3305-2002 from Human Metabolome Technologies America Inc. (HMT). The buffer solution used was a buffer solution of HMT Cat. No. 3301-1001. The measurement was performed at an applied voltage of +27 kV at a capillary temperature of 20°C. The sample was injected at 50 mbar over 10 seconds by use of the pressurization method.

2) Analysis conditions for time-of-flight mass spectrometer (Q-TOF MS)

A cation mode was used. The ionization voltage was set to 4 kV, the fragmentor voltage was set to 80 V, the skimmer voltage was set to 50 V, and the octRFV voltage was set to 650 V. The dry gas used was nitrogen, and its temperature and pressure were set to 300°C and 5 psig, respectively. The sheath solution used was a sheath solution of HMT Cat. No. H3301-1020. The reference masses were set to m/z 65.059706 and m/z 622.08963.

(c-2) Anionic metabolite substance measurement conditions

1) Measurement equipment

Agilent 6210 TOF system equipped with Agilent 1600 CE system (manufactured by Agilent Technologies, Inc.) was used in the measurement of anionic metabolite substances. The capillary and its temperature used had the same settings as those for the cation. The buffer solution used was a buffer solution of HMT Cat. No. H3302-1021. The applied voltage was 30 kV. The sample was injected at 50 mbar over 25 seconds by use of the pressurization method.

2) Analysis conditions for time-of-flight mass spectrometer (TOF MS)

A new anion mode was used. The ionization voltage was set to 3.5 kV, the fragmentor voltage was set to 125 V, the skimmer voltage was set to 50 V, and the octRFV voltage was set to 175 V. The same dry gas and sheath solution as those for the cation were used under the same conditions thereas. The reference masses were set to m/z 51.013854 and m/z 680.035541.

(c-3) Data processing

In order to obtain information on m/z, migration times (MT), and peaks including peak regions, raw data on peaks found by CE-Q-TOF MS or CE-TOF MS was processed using Master Hands automatic integration software version 2.0 (manufactured by Keio University). All the peaks were found with the software, and noise was removed to generate data matrix including the annotation and relative peak areas of metabolites. The peaks were annotated with metabolite names estimated from the HMT metabolite database on the basis of m/z obtained from CE and MT obtained from TOF MS. The conditions of MT, m/z, and the minimum S/N ratio for annotating anion peaks were set to 1.5 minutes, 50 ppm, and 20, respectively, while those for cations were set to 0.5 minutes, 50 ppm, and 20, respectively.

The relative concentration of each annotated metabolite was calculated by dividing the peak area of the metabolite by the area of ISC1 (cation) or ISA1 (anion).

In the CE-Q-TOF MS and CE-TOF MS analysis, anti-tumor effects on each individual patient were masked for analyzers.

A list of the processed peaks was output for further statistical analysis. In the statistical analysis, the relative concentrations of each annotated metabolite measured in duplicate were averaged.

(d) Statistical analysis

Clinical and metabolomics data processing and statistical analysis employed JMP 64-Bit Edition version 12 (manufactured by SAS Institute Inc.) in Microsoft Windows 7.

In this trial, 68 serum samples each before the start of treatment of this trial, after implementation of cycle 1, and after implementation of cycle 2 were obtained from 68 patients. Data on metabolites obtained from 68 serum samples each after implementation of cycle 1 and after implementation of cycle 2 was used for studying predictors of therapeutic effects.

In this trial, the maximum anti-tumor effect during the study treatment period was identified, and a therapeutic response group (responders) and a treatment non-response group (non-responders) were defined as follows.

- Responder (R): patient who exhibited complete response or partial response in terms of the maximum effect during the study treatment period as a result of diagnostic imaging by radiologists according to the RECIST criteria.
- Non-responder (N-R): patient who exhibited stable disease or progressive disease in terms of the maximum effect during the study treatment period as a result of diagnostic imaging by radiologists according to the RECIST criteria.

The chi-square test was used for confirming difference in patient background.

In order to study difference in each metabolite between the N-R group and the R group, nominal logistic analysis was comprehensively conducted on the measured metabolites, and metabolites which were significant in the whole model were used as candidate substances of markers for determining sensitivity to an anti-cancer agent early after the start of treatment with the anti-cancer agent.

Next, in order to establish effect prediction models based on the candidate substances of markers for determining sensitivity to an anti-cancer agent early after the start of treatment with the anti-cancer agent, a cutoff value was determined for each univariately significant metabolite from ROC curve, and binarization was carried out based on this value. Variables were selected by the step-up method with Bayesian information criterion (BIC) as an index in the step wise method using the binarized value. Multivariate nominal logistic regression analysis was conducted using the variables thus selected. Receiver-operator characteristics (ROC) were used for evaluating the effect prediction performance of the candidate substances of markers for determining sensitivity to an anti-cancer agent early after the start of treatment with the anti-cancer agent.

In order to evaluate actual clinical effects and effect prediction performance based on the prediction models, confusion matrix was prepared, and the prediction performance for the responders was evaluated from sensitivity, specificity and accuracy.

For the purpose of studying the prognosis prediction performance of the effect prediction models, residual overall survivals were estimated by use of the Kaplan-Meier method, and difference between the responders and the non-responders was studied by use of the log-rank test.

Further, from the concentrations of blood metabolites early after the start of treatment and the residual overall survival of each patient, metabolites for predicting prognosis were analyzed using COX proportional hazard model. In the statistical analysis described above, $p < 0.05$ was regarded as being significant.

(2) Results

(a) Substance which exhibited significant difference between R group and N-R group

As shown in Table 1, 29 patients were defined as N-R, and 39 patients were defined as R. No difference in patient background was seen between the R group and the N-R group.

[Table 1]

| | | N-R | R | *p* value |
|---|---|---|---|---|
| Sex | | | | |
| | Male | 17 | 21 | 0.8063 |
| | Female | 12 | 18 | |
| Age | | | | |
| | Media(years) | 64 | 63 | 0.1106 |
| | Range | 54-78 | 28-81 | |
| ECOG PS | | | | |
| | 0 | 25 | 29 | 0.3638 |
| | 1 | 4 | 10 | |
| Primary site | | | | |
| | Colon | 19 | 25 | 1.0000 |
| | Rectum | 10 | 14 | |
| Primary site | | | | |
| | Resected | 21 | 33 | 0.2413 |
| | Exist | 8 | 6 | |
| Baseline total tumor length (mm) | | | | |
| | Media | 90.7 | 97.9 | 0.6204 |
| | Range | 16.4-261.0 | 12.2-247.3 | |
| Histological type | | | | |
| | Well-differentiated | 8 | 8 | 0.533 |
| | Moderately differentiated | 17 | 26 | |
| | Poorly differentiated | 2 | 4 | |
| | Mucinous | 2 | 0 | (Well-differentiated vs. Moderately differentiated vs. others) |
| | Mixture of well-differenciated and moderately differentiated | 0 | 1 | |
| Number of metastatic organs | | | | |
| | 1 | 12 | 18 | 0.8063 |
| | $\geq 2$ | 17 | 21 | |
| Adjuvant chemotherapy | | | | |
| | Yes | 28 | 36 | 0.6306 |
| | No | 1 | 3 | |

**N-R, Non Responder. R, Responder**
**ECOG PS, Eastern Cooperative Oncology Group Performance status.**

Nominal logistic analysis using response to treatment (whether to be a responder or a non-responder) as objective variables was comprehensively conducted on metabolites with a detection rate of 25% or more among 480 metabolites whose expression was found in serum samples obtained before this study treatment. As a result, as shown in Table 2, 12 metabolites were significant in cycle 1, and 9 metabolites were significant in cycle 2. Figures 1 and 2 show the distributions of responders and non-responders for each metabolite.

[Table 2]

| Metabolite | Cycle 1 | Cycle 2 |
|---|---|---|
| 2-Aminoadipic acid | 0.0278 | |
| 2-Aminobutyric acid | | 0.0401 |
| 2-Cyaropyridine | 0.0044 | |
| 5-Oxoproline | | 0.0182 |
| 6-Aminohexanoic acid | 0.0204 | 0.0089 |
| Adenosine | | 0.017 |
| Aspartic acid | | 0.0175 |
| Betonicine | 0.01 | |
| Carbachol | 0.0465 | |
| Cysteine-glutathione disulphide | 0.0166 | |
| Dopamine | | 0.0072 |
| gamma-Glu-Cys | 0.0289 | |
| Oxidised Glutathione (GSSG) | 0.0158 | |
| Hypotaurine | | 0.024 |
| Methionine sulfoxide | | 0.0479 |
| N6-Methyl-2'-deoxyadenosine | 0.0371 | |
| Nomega-Methyltryptamine | 0.0161 | |
| Phenyl phosphate | 0.0333 | |
| Proline | | 0.0369 |
| Ribulose 5-phosphate | 0.0228 | |
| | (p-value) | |

Responder and non-responder thresholds were determined for the metabolites which were significant as a result of comprehensively conducting nominal logistic analysis on a cycle basis on the metabolites detected in 25% or more patients, and binarization was carried out based on these thresholds. In this respect, as for prediction performance, ROC AUC, responder/non-responder prediction accuracy, sensitivity, specificity and accuracy were determined. The respective results are shown in Table 3. The cutoff value of each substance was as follows: the metabolites from cycle 1 were 2AMAD $(2.091 \times 10^{-2} \leq)$ ; 2CYPR $(3.444 \times 10^{-3} \leq)$ ; 6AHXA $(7.175 \times 10^{-3} \leq)$ ; BETNC $(1.589 \times 10^{-2} \leq)$ ; CARB $(\leq 8.472 \times 10^{-3})$ ; CSSG $(2.198 \times 10^{-2} \leq)$ ; GGLCY $(7.389 \times 10^{-3} \leq)$ ; GSSG $(5.606 \times 10^{-4} \leq)$ ; N6MDA $(\leq 1.208 \times 10^{-3})$; NOMTR $(\leq 7.006 \times 10^{-2})$; PHEP $(\leq 1.801 \times 10^{-2})$ ; and RIB5P $(\leq 3.005 \times 10^{-3})$. The metabolites from cycle 2 were 2ABA $(2.507 \times 10^{-1} \leq)$; 5OPRO $(1.843 \times 10^{-1} \leq)$ ; 6AHXA $(4.568 \times 10^{-3} \leq)$ ; ADEN $(2.305 \times 10^{-2} \leq)$ ; ASP $(\leq 1.170 \times 10^{-1})$; DOPM $(5.606 \times 10^{-4} \leq)$ ; HYPT $(\leq 1.768 \times 10^{-2})$ ; METSF $(3.836 \times 10^{-2} \leq)$ ; and PRO $(2.9876 \leq)$. CSSG in cycle 1 exhibited particularly favorable AUC $(0.7 \leq)$. 2ABA in cycle 1 or PRO in cycle 2, alone, exhibited 100% specificity and positive predictive value.

[Table 3]

| Cycle | Metabolites | Responder Cut-off values | ROC AUC | Responder Predictive value | Non-responder Predictive value | Sensitivity | Specificity | Accuracy |
|---|---|---|---|---|---|---|---|---|
| Cycle 1 | 2AMAD | $2.091 \times 10^{-2} \leq$ | 0.63 | 100.0 | 50.0 | 25.6 | 100.0 | 57.4 |
| | 2CYPR | $3.444 \times 10^{-3} \leq$ | 0.62 | 38.5 | 86.2 | 78.9 | 51.0 | 58.8 |
| | 6AHXA | $7.175 \times 10^{-3} \leq$ | 0.66 | 38.5 | 93.1 | 88.2 | 52.9 | 61.8 |
| | BETNC | $1.589 \times 10^{-2} \leq$ | 0.64 | 30.8 | 96.6 | 92.3 | 50.9 | 58.8 |
| | CARB | $\leq 8.472 \times 10^{-3}$ | 0.60 | 79.5 | 41.4 | 64.6 | 60.0 | 63.2 |
| | CSSG | $2.198 \times 10^{-2} \leq$ | 0.70 | 53.8 | 86.2 | 84.0 | 58.1 | 67.6 |
| | GGLCY | $7.389 \times 10^{-3} \leq$ | 0.67 | 61.5 | 72.4 | 75.0 | 58.3 | 66.2 |
| | GSSG | $5.606 \times 10^{-4} \leq$ | 0.59 | 20.5 | 96.6 | 88.9 | 47.5 | 52.9 |
| | N6MDA | $\leq 1.208 \times 10^{-3}$ | 0.65 | 82.1 | 48.3 | 68.1 | 66.7 | 67.6 |
| | NOMTR | $\leq 7.006 \times 10^{-2}$ | 0.63 | 92.3 | 34.5 | 65.5 | 76.9 | 67.6 |
| | PHEP | $\leq 1.801 \times 10^{-2}$ | 0.58 | 92.3 | 24.1 | 62.1 | 70.0 | 63.2 |
| | RIB5P | $\leq 3.005 \times 10^{-3}$ | 0.65 | 92.3 | 37.9 | 66.7 | 78.6 | 69.1 |
| Cycle 2 | 2ABA | $2.507 \times 10^{-1} \leq$ | 0.65 | 41.0 | 89.3 | 84.2 | 52.1 | 61.2 |
| | 5OPRO | $1.843 \times 10^{-1} \leq$ | 0.64 | 30.8 | 96.4 | 92.3 | 50.0 | 58.2 |
| | 6AHXA | $4.568 \times 10^{-3} \leq$ | 0.64 | 56.4 | 71.4 | 73.3 | 54.1 | 62.7 |
| | ADEN | $2.305 \times 10^{-2} \leq$ | 0.64 | 92.3 | 35.7 | 66.7 | 76.9 | 68.7 |
| | ASP | $\leq 1.170 \times 10^{-1}$ | 0.65 | 89.7 | 39.3 | 67.3 | 73.3 | 68.7 |
| | DOPM | $5.606 \times 10^{-4} \leq$ | 0.63 | 33.3 | 92.9 | 86.7 | 50.0 | 58.2 |
| | HYPT | $\leq 1.768 \times 10^{-2}$ | 0.67 | 79.5 | 53.6 | 70.5 | 65.2 | 68.7 |
| | METSF | $3.836 \times 10^{-2} \leq$ | 0.62 | 28.2 | 96.4 | 91.7 | 49.1 | 56.7 |
| | PRO | $2.9876 \leq$ | 0.64 | 100.0 | 28.6 | 66.1 | 100.0 | 70.1 |

(b) Calculation of model for determining anti-cancer agent sensitivity

Models for determining sensitivity to an anti-cancer agent were prepared by sifting through explanatory variables by use of the step wise method. The results are shown in Table 4. The models were formed using 5 metabolites 2AMAD, BETNC, CSSG, N6MDA and RIB5P in cycle 1 and using 6 metabolites 2ABA, ASP, DOPM, HYPT, METSF and PRO in cycle 2.

[Table 4]

| | Metabolites | Responder Cut-off value | Parameter | | Equation of Responder probability |
|---|---|---|---|---|---|
| | | | Non-responder | Responder | |
| Cycle 1 | 2AMAD | $2.091 \times 10^{-2} \leq$ | 16.2688 | - 16.2688 | p = 1/[1+Exp(-16.4681 + Parameter : 2AMAD + Parameter : BETNC + Parameter : CSSG + Parameter : N6MDA + Parameter : RIB5P)] |
| | BETNC | $1.589 \times 10^{-2} \leq$ | 8.6560 | - 8.6560 | |
| | CSSG | $2.198 \times 10^{-2} \leq$ | 1.4372 | - 1.4372 | |
| | N6MDA | $\leq 1.208 \times 10^{-3}$ | 8.6658 | - 8.6658 | |
| | RIB5P | $\leq 3.005 \times 10^{-3}$ | 1.3451 | - 1.3451 | |
| Cycle 2 | 2ABA | $2.507 \times 10^{-1} \leq$ | 2.3059 | - 2.3059 | p = 1/[1+Exp(6.4305 + Parameter :2ABA + Parameter :ASP + Parameter :DOPM + Parameter :HYPT + Parameter :METSF + Parameter :PRO)] |
| | ASP | $\leq 1.170 \times 10^{-1}$ | 1.8154 | - 1.8154 | |
| | DOPM | $5.606 \times 10^{-4} \leq$ | 1.6345 | - 1.6345 | |
| | HYPT | $\leq 1.768 \times 10^{-2}$ | 2.4113 | - 2.4113 | |
| | METSF | $3.836 \times 10^{-2} \leq$ | 1.5555 | - 1.5555 | |
| | PRO | $2.9876 \leq$ | 9.0794 | - 9.0794 | |

The models for determining sensitivity to an anti-cancer agent (cycle 1 model and cycle 2 model) were as shown in the following equations (1) and (2), respectively.

## Cycle 1 model

$$p = \frac{1}{1 + e^{(-16.4681+(2AMAD)+(BETNC)+(CSSG)+(N6MDA)+(RIB5P))}} \qquad (1)$$

wherein 2AMAD represents -16.2688 when a measurement result about 2AMAD ise equal to or more than a cutoff value, and 16.2688 when the measurement result is less than the cutoff value; BETNC represents -8.6560 when a measurement result about BETNC is equal to or more than a cutoff value, and 8.6560 when the measurement result is less than the cutoff value; CSSG represents -1.4372 when a measurement result about CSSG is equal to or more than a cutoff value, and 1.4372 when the measurement result is less than the cutoff value; N6MDA represents 8.6658 when a measurement result about N6MDA is more than a cutoff value, and -8.6658 when the measurement result is equal to or less than the cutoff value; RIB5P represents 1.3451 when a measurement result about RIB5P is more than a cutoff value, and -1.3451 when the measurement result is equal to or less than the cutoff value; and the cutoff value is 2.091 $\times$ 10$^{-2}$ for 2AMAD, 1.589 $\times$ 10$^{-2}$ for BETNC, 2.198 $\times$ 10$^{-2}$ for CSSG, 1.208 $\times$ 10$^{-3}$ for N6MDA, and 3.005 $\times$ 10$^{-3}$ for RIB5P.

## Cycle 2 model

$$p = \frac{1}{1 + e^{(6.4305+(2ABA)+(ASP)+(DOPM)+(HYPT)+(METSF)+(PRO))}} \qquad (2)$$

wherein 2ABA represents -2.3059 when a measurement result about CSSG is equal to or more than a cutoff value, and 2.3059 when the measurement result is less than the cutoff value; ASP represents 1.8154 when a measurement result about ASP is more than a cutoff value, and -1.8154 when the measurement result is equal to or less than the cutoff value; DOPM represents -1.6345 when a measurement result about DOPM is equal to or more than a cutoff value, and 1.6345 when the measurement result is less than the cutoff value; HYPT represents 2.4113 when a measurement result about HYPT is more than a cutoff value, and - 2.4113 when the measurement result is equal to or less than the cutoff value; METSF represents -1.5555 when a measurement result about METSF is equal to or more than a cutoff value, and 1.5555 when the measurement result is less than the cutoff value; PRO represents -9.0794 when a measurement result about PRO is equal to or more than a cutoff value, and 9.0794 when the measurement result is less than the cutoff value; and the cutoff value is $2.507 \times 10^{-1}$ for 2ABA, $1.170 \times 10^{-1}$ for ASP, $5.606 \times 10^{-4}$ for DOPM, $1.768 \times 10^{-2}$ for HYPT, $3.836 \times 10^{-2}$ for METSF, and 2.9876 for PRO.

The cycle 1 model and the cycle 2 model were equations to determine whether or not a patient is a responder. The p value represents a probability that a patient is a responder. When the p value is more than 0.5, the patient is confirmed to be a responder.

AUC of the ROC curve of the cycle 1 model was 0.94 (Figure 3a). Its sensitivity, specificity, positive predictive value and negative predictive value were 100%, 65.5%, 79.6% and 100%, respectively, as shown in Table 5.

AUC of the ROC curve of the cycle 2 model was 0.97 (Figure 3b). Its sensitivity, specificity, positive predictive value and negative predictive value were 92.3%, 85.7%, 90.0% and 88.9%, respectively, as shown in Table 5.

[Table 5]

| | | | Predicted response | | | Predictive value (%) | | Sensitivity (%) | Specificity (%) | Accuracy (%) |
|---|---|---|---|---|---|---|---|---|---|---|
| | | | Non-responder | Responder | Total | Non-responder | Responder | | | |
| Cycle 1 | Actual response | Non-responder | 19 | 10 | 29 | 100 | 79.6 | 100 | 65.5 | 85.3 |
| | | Responder | 0 | 39 | 39 | | | | | |
| | | Total | 19 | 49 | 68 | | | | | |
| Cycle 2 | Actual response | Non-responder | 24 | 4 | 28 | 88.9 | 90.0 | 92.3 | 85.7 | 89.6 |
| | | Responder | 3 | 36 | 39 | | | | | |
| | | Total | 27 | 40 | 67 | | | | | |

(c) OS prediction performance of cycle 1 model and cycle 2 model

In order to verify the predictability of OS by the cycle 1 model and the cycle 2 model, patients were divided into a group confirmed to be a responder (R group) and a group confirmed to be a non-responder (N-R group) on the basis of the metabolites before the start of treatment using the models. A Kaplan-Meier curve was drawn to confirm whether OS would differ between the R group and the N-R group. OS was compared between the R group and the N-R group by the log-rank test.

As a result, for both the cycle 1 model and the cycle 2 model, the R group had significantly longer OS than that of the N-R group (p = 0.0008 and p = 0.0020, respectively). The results showed the usefulness of these metabolite models (Figures 4a and 4b).

(d) Analysis of OS using COX proportional hazard model of metabolite

Metabolites were comprehensively analyzed using COX proportional hazard model on the basis of survival periods from the days of administration in cycle 1 and cycle 2. As a result, the hazard ratios of significant metabolites and their 95% confidence intervals are shown in Figures 5a and 5b. It was found in cycle 1 that the higher the blood concentrations of BEZIZ, CSSG, GLC3P, GLYLE and QUINA, the longer the survival period while the higher the blood concentrations of DDNA, GUSA and OCTA, the shorter the survival period.

It was found in cycle 2 that the higher the blood concentrations of 2AMAD, ALA, BEZIZ, CSSG, GGLCY, GLCOA, GLU, GLTA, GLC3P, LEU, LYS, N6ALY, QUINA, THR and VAL, the longer the survival period while the higher the blood concentrations of 10HDA, CHOA and PIPEC, the shorter the survival period.

**Claims**

1. A marker for determining sensitivity to an anti-cancer agent early after the start of treatment with the anti-cancer agent, the anti-cancer agent comprising oxaliplatin or a salt thereof, fluorouracil or a salt thereof, and levofolinate or a salt thereof, the marker comprising one or more molecules selected from the group consisting of cysteine-glutathione disulphide (CSSG), 2-aminoadipic acid (2AMAD), betonicine (BETNC), N6-methyl-2'-deoxyadenosine (N6MDA), ribulose 5-phosphate (RIB5P), 6-aminohexanoic acid (6AHXA), aspartic acid (ASP), hypotaurine (HYPT), 2-aminobutyric acid (2ABA), dopamine (DOPM), methionine sulfoxide (METSF), proline (PRO), gamma-glutamylcysteine (GGLCY), oxidized glutathione (GSSG), N-omega-methyltryptamine (NOMTR), phenyl phosphate (PHEP), 2-cyanopyridine (2CYPR), carbachol (CARB), 5-oxoproline (5OPRO) and adenosine (ADEN).

2. The marker according to claim 1, wherein the anti-cancer agent further comprises bevacizumab.

3. A method for determining sensitivity to an anti-cancer agent early after the start of treatment with the anti-cancer agent, the anti-cancer agent comprising oxaliplatin or a salt thereof, fluorouracil or a salt thereof, and levofolinate or a salt thereof, the method comprising measuring an amount of one or more molecules selected from the group consisting of CSSG, 2AMAD, BETNC, N6MDA, RIB5P, 6AHXA, ASP, HYPT, 2ABA, DOPM, METSF, PRO, GGLCY, GSSG, NOMTR, PHEP, 2CYPR, CARB, 5OPRO and ADEN in a biological sample derived from a cancer patient who has received at least one cycle of treatment with the anti-cancer agent comprising oxaliplatin or a salt thereof, fluorouracil or a salt thereof, and levofolinate or a salt thereof.

4. The determination method according to claim 3, further comprising determining the sensitivity of the cancer patient to the anti-cancer agent by comparing a measurement result with a control level.

5. The determination method according to claim 4, wherein: the cancer patient is a patient who has received one cycle of treatment with the anti-cancer agent; the amount of one or more molecules selected from the group consisting of 2AMAD, 2CYPR, 6AHXA, BETNC, CARB, CSSG, GGLCY, GSSG, N6MDA, NOMTR, PHEP and RIB5P is measured; the control level is a cutoff value of a responder; and the cutoff value is $2.091 \times 10^{-2} \le$ for 2AMAD, $3.444 \times 10^{-3} \le$ for 2CYPR, $7.175 \times 10^{-3} \le$ for 6AHXA, $1.589 \times 10^{-2} \le$ for BETNC, $\le 8.472 \times 10^{-3}$ for CARB, $2.198 \times 10^{-2} \le$ for CSSG, $7.389 \times 10^{-3} \le$ for GGLCY, $5.606 \times 10^{-4} \le$ for GSSG, $\le 1.208 \times 10^{-3}$ for N6MDA, $\le 7.006 \times 10^{-2}$ for NOMTR, $\le 1.801 \times 10^{-2}$ for PHEP, and $\le 3.005 \times 10^{-3}$ for RIB5P.

6. The determination method according to claim 3, wherein the cancer patient is a patient who has received one cycle of treatment with the anti-cancer agent, and the method further comprises calculating a probability (p) of a responder according to the following equation (1) to determine whether or not the cancer patient is the responder:

$$p = \frac{1}{1 + e^{(-16.4681 + (2AMAD) + (BETNC) + (CSSG) + (N6MDA) + (RIB5P))}} \qquad (1)$$

wherein 2AMAD represents -16.2688 when a measurement result about 2AMAD is equal to or more than a cutoff value, and 16.2688 when the measurement result is less than the cutoff value; BETNC represents -8.6560 when a measurement result about BETNC is equal to or more than a cutoff value, and 8.6560 when the measurement result is less than the cutoff value; CSSG represents -1.4372 when a measurement result about CSSG is equal to or more than a cutoff value, and 1.4372 when the measurement result is less than the cutoff value; N6MDA represents -8.6658 when a measurement result about N6MDA is equal to or less than a cutoff value, and 8.6658 when the measurement result is more than the cutoff value; RIB5P represents -1.3451 when a measurement result about RIB5P is equal to or less than a cutoff value, and 1.3451 when the measurement result is more than the cutoff value; and the cutoff value is $2.091 \times 10^{-2}$ for 2AMAD, $1.589 \times 10^{-2}$ for BETNC, $2.198 \times 10^{-2}$ for CSSG, $1.208 \times 10^{-3}$ for N6MDA, and $3.005 \times 10^{-3}$ for RIB5P.

7. The determination method according to claim 4, wherein the cancer patient is a patient who has received two cycles of treatment with the anti-cancer agent; the amount of one or more molecules selected from the group consisting of 2ABA, 5OPRO, 6AHXA, ADEN, ASP, DOPM, HYPT, METSF and PRO is measured; the control level is a cutoff value of a responder; and the cutoff value is $2.507 \times 10^{-1} \le$ for 2ABA, $1.843 \times 10^{-1} \le$ for 5OPRO, $4.568 \times 10^{-3} \le$ for 6AHXA, $2.305 \times 10^{-2} \le$ for ADEN, $\le 1.170 \times 10^{-1}$ for ASP, $5.606 \times 10^{-4} \le$ for DOPM, $\le 1.768 \times 10^{-2}$ for HYPT, $3.836 \times 10^{-2} \le$ for METSF, and $2.9876 \le$ for PRO.

8. The determination method according to claim 3, wherein the cancer patient is a patient who has received two cycles of treatment with the anti-cancer agent, and the method further comprises calculating a probability (p) of a responder according to the following equation (2) to determine whether or not the cancer patient is the responder:

$$p = \frac{1}{1 + e^{(6.4305+(2ABA)+(ASP)+(DOPM)+(HYPT)+(METSF)+(PRO))}} \quad (2)$$

wherein 2ABA represents -2.3059 when a measurement result about 2ABA is equal to or more than a cutoff value, and 2.3059 when the measurement result is less than the cutoff value; ASP represents -1.8154 when a measurement result about ASP is equal to or less than a cutoff value, and 1.8154 when the measurement result is more than the cutoff value; DOPM represents -1.6345 when a measurement result about DOPM is equal to or more than a cutoff value, and 1.6345 when the measurement result is less than the cutoff value; HYPT represents -2.4113 when a measurement result about HYPT is equal to or less than a cutoff value, and 2.4113 when the measurement result is more than the cutoff value; METSF represents -1.5555 when a measurement result about METSF is equal to or more than a cutoff value, and 1.5555 when the measurement result is less than the cutoff value; PRO represents -9.0794 when a measurement result about PRO is equal to or more than a cutoff value, and 9.0794 when the measurement result is less than the cutoff value; and the cutoff value is $2.507 \times 10^{-1}$ for 2ABA, $1.170 \times 10^{-1}$ for ASP, $5.606 \times 10^{-4}$ for DOPM, $1.768 \times 10^{-2}$ for HYPT, $3.836 \times 10^{-2}$ for METSF, and 2.9876 for PRO.

9. The determination method according to any one of claims 3 to 8, wherein the anti-cancer agent further comprises bevacizumab.

10. A marker for predicting prognosis early after the start of treatment with an anticancer agent, the anti-cancer agent comprising oxaliplatin or a salt thereof, fluorouracil or a salt thereof, and levofolinate or a salt thereof, the marker comprising one or more molecules selected from the group consisting of CSSG, glycerol-3-phosphate (GLC3P), quinic acid (QUINA), benzimidazole (BEZIZ), glycylleucine (GLYLE), GGLCY, dodecanedioic acid (DDNA), guanidinosuccinic acid (GUSA), octanoic acid (OCTA), 10-hydroxydecanoic acid (10HDA), 2AMAD, alanine (ALA), cholic acid (CHOA), gluconic acid (GLCOA), glutamic acid (GLU), glutaric acid (GLTA), leucine (LEU), lysine (LYS), N6-acetyllysine (N6ALY), pipecolic acid (PIPEC), threonine (THR) and valine (VAL).

11. The marker according to claim 10, wherein the anti-cancer agent further comprises bevacizumab.

12. A method for predicting prognosis early after the start of treatment with an anti-cancer agent, the anti-cancer agent comprising oxaliplatin or a salt thereof, fluorouracil or a salt thereof, and levofolinate or a salt thereof, the method comprising measuring an amount of one or more molecules selected from the group consisting of CSSG, GLC3P, QUINA, BEZIZ, GLYLE, GGLCY, DDNA, GUSA, OCTA, 10HDA, 2AMAD, ALA, CHOA, GLCOA, GLU, GLTA, LEU, LYS, N6ALY, PIPEC, THR and VAL in a biological sample derived from a cancer patient who has received at least one cycle of treatment with the anti-cancer agent comprising oxaliplatin or a salt thereof, fluorouracil or a salt thereof, and levofolinate or a salt thereof.

13. The prognosis prediction method according to claim 12, wherein the anti-cancer agent further comprises bevacizumab.

14. A kit for carrying out a determination method according to any one of claims 3 to 9, the kit comprising a protocol for measuring an amount of one or more molecules selected from the group consisting of CSSG, 2AMAD, BETNC, N6MDA, RIB5P, 6AHXA, ASP, HYPT, 2ABA, DOPM, METSF, PRO, GGLCY, GSSG, NOMTR, PHEP, 2CYPR, CARB, 5OPRO and ADEN in a biological sample derived from a cancer patient who has received at least one cycle of treatment with the anti-cancer agent comprising oxaliplatin or a salt thereof, fluorouracil or a salt thereof, and levofolinate or a salt thereof.

15. A kit for carrying out a prognosis prediction method according to claim 12 or 13, the kit comprising a protocol for measuring an amount of one or more molecules selected from the group consisting of CSSG, GLC3P, QUINA, BEZIZ, GLYLE, GGLCY, DDNA, GUSA, OCTA, 10HDA, 2AMAD, ALA, CHOA, GLCOA, GLU, GLTA, LEU, LYS, N6ALY, PIPEC, THR and VAL in a biological sample derived from a cancer patient who has received at least one cycle of treatment with the anti-cancer agent comprising oxaliplatin or a salt thereof, fluorouracil or a salt thereof, and levofolinate or a salt thereof.

[Figure 1]

[Figure 2]

[Figure 3]

[Figure 4]

[Figure 5]

a. Residual OS hazard ratio (cycle 1)

Metabolites
Benzimidazole
Cysteine-glutathione disulphide
Dodecanedioic acid
Glycerol-3-phosphate
Gly-Leu
Guanidinosuccinic acid
Octanoic acid
Quinic acid

0.001    0.01    0.1    1    10    100

b. Residual OS hazard ratio (cycle 2)

Metabolites
10-Hydroxydecanoic acid
2-Aminoadipic acid
Alanine
Benzimidazole
Cholic acid
Cysteine-glutathione disulphide
gamma-Glu-Cys
Gluconic acid
Glutamic acid
Glutaric acid
Glycerol-3-phosphate
Leucine
Lysine
N6-Acetyllysine
Pipecolic acid
Quinic acid
Threonine
Valine

0.001    0.01    0.1    1    10    100    1000    10000    100000

## INTERNATIONAL SEARCH REPORT

| International application No. |
| --- |
| PCT/JP2019/037733 |

### A. CLASSIFICATION OF SUBJECT MATTER

A61K 31/282(2006.01)i; A61K 31/513(2006.01)i; A61K 31/519(2006.01)i; A61K 39/395(2006.01)i; A6IP 35/00(2006.01)i; A6IP 43/00(2006.01)i; G01N 33/50(2006.01)i; G01N 33/68(2006.01)i

FI: G01N33/68; G01N33/50 Z; A61K39/395 N; A61K39/395 T; A61K31/282; A61K31/513; A6IK31/519; A61P35/00; A61P43/00 121

According to International Patent Classification (IPC) or to both national classification and IPC

### B. FIELDS SEARCHED

Minimum documentation searched (classification system followed by classification symbols)

A61K31/282; A61K31/513; A61K31/519; A61K39/395; A61P35/00; A61P43/00; G01N33/50; G01N33/68

Documentation searched other than minimum documentation to the extent that such documents are included in the fields searched

| | |
| --- | --- |
| Published examined utility model applications of Japan | 1922-1996 |
| Published unexamined utility model applications of Japan | 1971-2020 |
| Registered utility model specifications of Japan | 1996-2020 |
| Published registered utility model applications of Japan | 1994-2020 |

Electronic data base consulted during the international search (name of data base and, where practicable, search terms used)

JSTPlus/ JMEDPlus/ JST7580 (JDreamIII); CAplus/MEDLINE/EMBASE/BIOSIS/WPIDS (STN)

### C. DOCUMENTS CONSIDERED TO BE RELEVANT

| Category* | Citation of document, with indication, where appropriate, of the relevant passages | Relevant to claim No. |
| --- | --- | --- |
| A | WO 2011/052750 A1 (KEIO GIJUKU) 05.05.2011 (2011-05-05) see entire text, all drawings | 1-9, 14 |
| A | WO 2011/052749 A1 (KEIO GIJUKU) 05.05.2011 (2011-05-05) see entire text, all drawings | 1-9, 14 |
| A | WO 2009/096196 A1 (KEIO GIJUKU) 06.08.2009 (2009-08-06) see entire text, all drawings | 1-9, 14 |

☒ Further documents are listed in the continuation of Box C.      ☒ See patent family annex.

| | |
| --- | --- |
| * Special categories of cited documents: | "T" later document published after the international filing date or priority date and not in conflict with the application but cited to understand the principle or theory underlying the invention |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 15 January 2020 (15.01.2020) | 28 January 2020 (28.01.2020) |

| Name and mailing address of the ISA/ | Authorized officer |
| --- | --- |
| Japan Patent Office | |
| 3-4-3, Kasumigaseki, Chiyoda-ku, | |
| Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/037733 |

C (Continuation).   DOCUMENTS CONSIDERED TO BE RELEVANT

| Category* | Citation of document, with indication, where appropriate, of the relevant passages | Relevant to claim No. |
| --- | --- | --- |
| A | JP 2010-215646 A (MENTAL HEALTH RESEARCH INST OF VICTORIA) 30.09.2010 (2010-09-30) see entire text, all drawings | 1-9, 14 |
| E, X | WO 2019/188446 A1 (KEIO GIJUKU) 03.10.2019 (2019-10-03) entire text, all drawings in particular, see claims, etc. | 1-9, 14 |
| P, X | WO 2018/181759 A1 (KEIO GIJUKU) 04.10.2018 (2018-10-04) entire text, all drawings, in particular, see claims, etc. | 1-9, 14 |

Form PCT/ISA/210 (continuation of second sheet) (January 2015)

## INTERNATIONAL SEARCH REPORT

| International application No. |
| --- |
| PCT/JP2019/037733 |

**Box No. II        Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:
See extra sheet

3. ☐ Claims Nos.:
because they are dependent claims and are not drafted in accordance with the second and third sentences of Rule 6.4(a).

**Box No. III        Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: Since the applicant did not pay the fees necessary for additional search before the deadline, this ISR was written for the claims relating to the invention set forth in claims at first. A marker, cysteine-glutathione disulphide (CSSG), set forth at first in the invention in claims 1-9 and 14 is used as a marker for determining sensitivity at early stage after the initiation of treatment using an anti-cancer agent, and the ISR was written for a determining method using the marker and a kit for performing the method.

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

**INTERNATIONAL SEARCH REPORT**

| International application No. |
|---|
| PCT/JP2019/037733 |

<Continuation of Box No. III>

As described below, the International Searching Authority acknowledged that there are two or more inventions in the present international application.

It is recognized that the technical features of the invention in claims 1-9 and 14 are: a marker for determining sensitivity at early stage after the initiation of treatment using an anti-cancer agent, the marker containing oxaliplatin or a salt thereof, fluorouracil or a salt thereof, and levofolinate or a salt thereof and comprising at least one molecule selected from 20 molecules consisting of cysteine-glutathione disulphide (CSSG), … and adenosine (ADEN); a determining method using the marker; and a kit for performing the method.

It is recognized that the technical features of the invention in claims 10-13 and 15 are: a marker for predicting prognosis at early stage after the initiation of treatment using an anti-cancer agent, the marker containing oxaliplatin or a salt thereof, fluorouracil or a salt thereof, and levofolinate or a salt thereof and comprising at least one molecule selected from 22 molecules consisting of CSSG, … and valine (VAL); a method for predicting prognosis using the marker at early stage after the initiation of treatment; and a kit for performing the method.

It is apparent that the technical feature of the invention in claims 1-9 and 14 is different from the technical feature of the invention in claims 10-13 and 15.

In addition, all of the 20 marker materials specified in the inventions in claims 1-9 and 14 are completely different materials with respect to structure, physiological activity, etc., and do not share a common structure. If the marker materials shared common properties and shared important structure elements essential for exhibiting the common properties, it would be determined that there is a single general inventive concept. However, since the specified 20 marker materials do not share a common structure as indicated above, the 20 marker materials are not considered to constitute a single general inventive concept.

Therefore, in the invention in claims 1-9 and 14, a separate invention is recognized to exist for each of the 20 marker materials.

In addition, 22 marker materials specified in the invention in claims 10-13 and 15 cannot be considered to constitute a single general inventive concept as above.

Furthermore, the prerequisite that determining sensitivity of an anti-cancer agent on the basis of a marker level is also well-known at the time of filing the present application (for example, see WO 2009/096196 A1 (KEIO GIJUKU) 06 August 2009).

Therefore, the inventions in claims 1-15 are recognized to include 42 inventions as described above.

Form PCT/ISA/210 (extra sheet) (January 2015)

| INTERNATIONAL SEARCH REPORT<br>Information on patent family members | | International application No.<br>PCT/JP2019/037733 | |
|---|---|---|---|
| Patent Documents<br>referred in the<br>Report | Publication Date | Patent Family | Publication Date |
| WO 2011/052750 A1 | 05 May 2011 | US 2012/0220618 A1<br>EP 2495561 A1<br>EP 3144670 A2<br>EP 3438656 A2 | |
| WO 2011/052749 A1 | 05 May 2011 | US 2012/0214831 A1<br>EP 2495569 A1<br>EP 3081941 A1 | |
| WO 2009/096196 A1 | 06 Aug. 2009 | US 2010/0323034 A1<br>EP 2241334 A1 | |
| JP 2010-215646 A | 30 Sep. 2010 | JP 2005-508333 A<br>JP 2013-234193 A<br>US 2005/0032708 A1<br>US 2013/0065961 A1<br>US 2018/0177839 A1<br>WO 2003/026684 A1<br>EP 1438063 A1 | |
| WO 2019/188446 A1 | 03 Oct. 2019 | (Family: none) | |
| WO 2018/181759 A1 | 04 Oct. 2018 | (Family: none) | |

Form PCT/ISA/210 (patent family annex) (January 2015)

## REFERENCES CITED IN THE DESCRIPTION

### Patent documents cited in the description

- WO 2009096189 A
- WO 2011052750 A
- WO 2012127984 A
- WO 2013125675 A
- JP 2017007980 A
- JP 2017014167 A

### Non-patent literature cited in the description

- *J Natl Cancer Inst.,* 02 February 2000, vol. 92 (3), 205-16
- *J Proteome Res.,* September 2003, vol. 2 (5), 488-94
- *Metabolomics,* March 2010, vol. 6 (1), 78-95
- *Metabolomics,* April 2013, vol. 9 (2), 444-453